# EUROPEAN PATENT APPLICATION

(11) **EP 4 413 864 A2**
(43) Date of publication of application: **14.08.2024**
(21) Application number: 24171712.3
(22) Date of filing: 03.10.2017
(51) Int. Cl.: A23C 3/02, A23C 9/20, A23L 2/38, A23L 2/46, A23L 3/02, A23L 3/16, A23L 3/22, A23C 3/033

(54) **METHODS AND SYSTEMS FOR GENERATING A STERILIZED HUMAN MILK PRODUCT**

(30) Priority: 04.10.2016 US 201662404097 P; 27.12.2016 US 201662439408 P; 18.04.2017 US 201762486884 P
(62) Divisional of application: 17859017.0
(71) Applicant: LactaLogics, Inc., Port St. Lucie, FL 34952 (US)
(72) Inventor: SNOW, Glenn, W., Reno, NV 89509 (US); SALTER, Laura, Reno, NV 89509 (US)
(74) Representative: HGF

(57) **Abstract**

Systems and methods for generating a sterilized human milk product from raw human milk, and sterilized human milk product compositions obtained thereby, are provided. The method includes sterilizing the milk using a countercurrent heating process. The temperature of the milk is raised to a target temperature for a duration of time that is refined to provide a sterilized human milk product with low overall bioburden while also maintaining nutritional quality. As such, the sterilized milk product is suitable for ingestion by premature infants.

## Description

### 1. CROSS REFERENCE TO RELATED APPLICATIONS

This application claims benefit to U.S. Provisional Application Number 62/404,097 filed on October 4, 2016, U.S. Provisional Application Number 62/439,408 filed on December 27, 2016, and U.S. Provisional Application Number 62/486,884 filed on April 18, 2017. The content of each of the above referenced applications is incorporated by reference in its entirety.

### 2. FIELD OF THE INVENTION

The present invention relates to a method for the continuous flow sterilization of human milk to generate a sterilized human milk product. More particularly, the present invention relates to a method for the reduction of *Bacillus Cereus* (*B. Cereus*) and *Clostridium Botulinum* (*C. Botulinum)* content in human milk by sterilization under conditions chosen to provide a sterilized human milk product suitable for remote consumption by infants, particularly premature infants.

### 3. BACKGROUND

The provision of human breast milk for remote consumption by an individual such as an adult, an adolescent, or an infant, requires the reduction in the content of pathogens, such as bacteria, viruses, mold, spores, and yeast to acceptable levels, with concomitant retention of the nutrition and biologically active agents within the milk that are beneficial for the individual when ingested.

A major risk in the supply of human breast milk for consumption is the presence of one or both of *B. Cereus* and *C. Botulinum* in the supply. *B. Cereus* originates from the human milk donor. Not all donors will be carriers, but the pooling of donor milk during production of a human milk product can result in more widespread contamination. *B. Cereus* is a heat resistant, spore forming, gram-positive bacterium that, when ingested, is harmful and results in infections of the bloodstream, lungs, and central nervous system which require treatment with antibiotics such as vancomycin, tobramycin, meropenem, and clindamycin. *C. Botulinum* causes botulism. Toxins produced by *C. Botulinum* can block nerve functions, resulting in various forms of paralysis and therefore can be a serious, potentially fatal, disease.

Many human milk banks use pasteurization during human milk processing. As applied to human milk, pasteurization typically involves heating the milk at 62.5°C for 30 minutes ("Holder pasteurization"). *See* Guidelines of the Establishment and Operation of a Donor Human Milk Bank, Human Milk Banking Association of North America, 2015. Methods that eliminate *B. Cereus* and *C*. *Botulinum* employ more extreme processing conditions. These extreme processing conditions, as well as the conditions employed for Holder pasteurization, often have a detrimental effect on the quality of the sterilized human milk. Namely, the process irreversibly damages nutritional proteins, lipids, and carbohydrates (e.g., oligosaccharides). Therefore, although the sterilized human milk is cleared of pathogens and is safe for consumption, the nutritional content is also substantially altered, such that the benefits rendered by consumption of the sterilized human milk are lost. Moreover, some processes used to reduce *B. Cereus* and *C*. *Botulinum* content include direct injection of heated steam into the milk, which risks introduction of contaminants and necessitates the downstream separation of water from the milk.

Accordingly, there is a need for a human milk sterilization process that reduces or eliminates harmful pathogens, such as *B. Cereus* and *C*. *Botulinum,* while also maintaining the nutritional qualities of the human milk.

### 4. SUMMARY

The present disclosure provides a method for the sterilization and generation of a sterilized human milk product. Raw human milk is provided from at least one donor. The raw human milk may have both *B. Cereus* and *C. Botulinum.* The raw human milk is provided to a closed in-line sterilization system to generate the sterilized human milk product. The raw human milk passes through at least one bacterial clarifier, and is further separated into a cream fraction and skim fraction. The skim fraction can then be further concentrated to generate a retentate. A standardized milk product is engineered by combining a portion of the cream fraction and a portion of the retentate to achieve desired concentrations of components and/or nutrients. In some embodiments, the standardized milk product is fortified with additional components and/or nutrients. The standardized milk product is further sterilized through a countercurrent heat exchange process. Rapidly elevating the temperature of the standardized milk product to a target temperature and holding it at the target temperature for a duration of time eliminates pathogen content while also maintaining nutritional quality of the sterilized milk product. Importantly, the standardized milk product of the present disclosure exhibits at least a 12 Log reduction of *C. Botulinum* content and a greater than 1000 Log reduction of *B. Cereus.* The standardized milk product is rendered safe for consumption by adults, adolescents, and in particular, for premature infants.

Accordingly, disclosed herein is a method of sterilizing human milk to produce a sterilized human milk product. The method comprises receiving, by a sterilizer located within a closed in-line system, as an input, a human milk sample; flowing the human milk sample through a tube of the sterilizer in a first direction at a first flow rate; heating the human milk sample in the tube by flowing heating fluid in a second direction at a second flow rate, wherein the heating fluid is in contact with an exterior surface of the tube; and cooling, by the sterilizer, the heated human milk sample.

In some embodiments, heating the human milk sample in the tube comprises: raising a temperature of the human milk sample to within a temperature range of 130° C and 150° C; and holding the temperature of the human milk sample within the temperature range for a duration of 3 to 15 seconds. In some embodiments, heating the human milk sample in the tube comprises: raising a temperature of the human milk sample to within a temperature range of 135° C and 145° C; and holding the temperature of the human milk sample within the temperature range for a duration of 6 to 14 seconds. In some embodiments, heating the human milk sample in the tube comprises: raising a temperature of the human milk sample to within a temperature range of 138 C and 142° C; and holding the temperature of the human milk sample within the temperature range for a duration of 8 to 13 seconds. In some embodiments, heating the human milk sample in the tube comprises: raising a temperature of the human milk sample to within a temperature range of 140° C and 141° C; and holding the temperature of the human milk sample within the temperature range for a duration of 12 to 13 seconds.

In various embodiments, a length of the portion of the tube and the first flow rate of the engineered human milk sample are each previously chosen to hold the temperature of the engineered human milk within the temperature range for the duration.

In some embodiments, the first tube of the pharmaceutical grade sterilizer has a diameter between 0.25 inches and 10 inches. In some embodiments, the first flow rate of the engineered human milk sample through the tube of the pharmaceutical grade sterilizer is between 0.25 gallons per minute and 25 gallons per minute. In some embodiments, heating the human milk sample in the tube comprises: preheating the human milk sample to a first temperature between 80°C and 100°C in a first portion of the tube; and heating the human milk sample to a second temperature between 130° C and 150° C in a second portion of the tube.

In some embodiments, the human milk sample received by the pharmaceutical grade sterilizer is non-homogenized. In some embodiments, the human milk sample is homogenized prior to being received by the sterilizer or homogenized after being cooled by the sterilizer.

In various embodiments, the method of sterilizing human milk to produce a sterilized human milk product comprises a step, prior to the sterilizer receiving the human milk sample, of eliminating pathogens from a raw human breast milk sample through one or more clarification processes. In some embodiments, the method further comprises: separating a clarified human milk sample into a cream sample and a skim sample; generating a retentate by performing a concentration process on the skim sample; and engineering a standardized human milk sample to produce an engineered human milk product by combining a portion of the cream sample with a portion of the obtained retentate. In some embodiments, the method of combining a portion of the cream sample with a portion of the obtained retentate comprises: detecting an initial characteristic of the cream sample; comparing the detected initial characteristic of the cream sample to a target characteristic for the engineered human milk product; and determining the portion of the cream sample and the portion of the obtained retentate based on the comparison.

In various embodiments the method further comprises a step subsequent to cooling of the heated engineered human milk, the step comprising packaging, through an aseptic process, the sterilized human milk product. In various embodiments, the sterilizer is one of a commercial grade sterilizer or a pharmaceutical grade sterilizer.

In some embodiments, the sterilized human milk product has at least 63 calories per 100 milliliters of the sterilized human milk product. In some embodiments, the sterilized human milk product has a total fat content of at least 3.0 g/100 mL (3 wt%).

In various embodiments, the sterilized human milk product retains above 88% of immunoglobulin A (IgA) in comparison to a raw human breast milk sample that the sterilized human milk product derives from. In various embodiments, the sterilized human milk product retains above 77% of immunoglobulin M (IgM) in comparison to a raw human breast milk sample that the sterilized human milk product derives from. In various embodiments, the sterilized human milk product retains above 93% of immunoglobulin G (IgG) in comparison to a raw human breast milk sample that the sterilized human milk product derives from. In various embodiments, the sterilized human milk product retains above 64% of anti-trypsin in comparison to a raw human breast milk sample that the sterilized human milk product derives from. In various embodiments, the sterilized human milk product retains above 81% of lactoferrin in comparison to a raw human breast milk sample that the sterilized human milk product derives from. In various embodiments, the sterilized human milk product retains above 78% of lysozyme in comparison to a raw human breast milk sample that the sterilized human milk product derives from. In various embodiments, the sterilized human milk product retains above 72% of lactalbumin in comparison to a raw human breast milk sample that the sterilized human milk product derives from. In various embodiments, the sterilized human milk product retains above 92% of Alpha Casein in comparison to a raw human breast milk sample that the sterilized human milk product derives from. In various embodiments, the sterilized human milk product retains above 92% of Beta Casein in comparison to a raw human breast milk sample that the sterilized human milk product derives from. In various embodiments, the sterilized human milk product retains above 92% of Kappa Casein in comparison to a raw human breast milk sample that the sterilized human milk product derives from. In other embodiment, the sterilized human milk product retains above 87% of osteopontin in comparison to a raw human breast milk sample that the sterilized human milk product derives from.

In various embodiments, the sterilized human milk product retains above 92% of total human milk oligosaccharides in comparison to a raw human breast milk sample that the sterilized human milk product derives from. In various embodiments, the sterilized human milk product retains above 90% of fucosylated human milk oligosaccharides in comparison to a raw human breast milk sample that the sterilized human milk product derives from. In various embodiments, the sterilized human milk product retains above 90% of 2'-fucosyllactose in comparison to a raw human breast milk sample that the sterilized human milk product derives from. In various embodiments, the sterilized human milk product retains above 90% of 3'-fucosyllactose in comparison to a raw human breast milk sample that the sterilized human milk product derives from. In various embodiments, the sterilized human milk product retains above 90% of sialylated human milk oligosaccharides in comparison to a raw human breast milk sample that the sterilized human milk product derives from. In various embodiments, the sterilized human milk product retains above 90% of non-fucosylated human milk oligosaccharides in comparison to a raw human breast milk sample that the sterilized human milk product derives from.

Accordingly, also disclosed herein is a closed, in-line processing system. The system comprises a sterilizer comprising: a tube configured to flow a human milk sample in a first direction at a first flow rate; and a container that a portion of the tube resides within, wherein the container is configured to flow a heating fluid in contact with an exterior surface of the portion of the tube in a second direction at a second flow rate to heat the human milk sample flowing through the portion of the first tube to a predetermined temperature for a duration.

In some embodiments, the predetermined temperature is within a temperature range of 130° C and 150° C and the predetermined duration is between 3 and 15 seconds. In some embodiments, the predetermined temperature is within a temperature range of 135° C and 145° C and the predetermined duration is between 6 and 14 seconds. In some embodiments, the predetermined temperature is within a temperature range of 138° C and 142° C and the predetermined duration is between 8 and 13 seconds. In some embodiments, the predetermined temperature is within a temperature range of 140° C and 141° C and the predetermined duration is between 12 and 13 seconds. In various embodiments, the tube of the sterilizer is a spiral tube, wherein the duration that the human milk sample is held at the predetermined temperature is dependent on a number of spirals of the spiral tube. In some embodiments, the first flow rate of the human milk sample through the tube of the pharmaceutical grade sterilizer is between 0.25 gallons per minute and 25 gallons per minute.

In various embodiments, the system further comprises: one or more bacterial clarifiers, each bacterial clarifier configured to eliminate pathogens from a raw human breast milk sample; a milk separator in fluid communication with the last in series of the at least one bacterial clarifier, the milk separator configured to separate a clarified human milk sample into a cream sample and a skim sample; a milk concentrator in fluid communication with the milk separator, the milk concentrator further configured to generate a retentate by performing a reverse osmosis process on the skim sample received from the milk separator; and a milk combiner configured to combine a portion of the cream sample from the milk separator and a portion of the retentate from the milk concentrator.

In some embodiments, the milk standardizer is in fluid communication with the sterilizer, and a homogenizer is not located in the closed, in-line processing system prior to the sterilizer. In some embodiments, the system is capable of undergoing a clean-in-place (CIP) method of cleaning. In some embodiments, the sterilizer is a pharmaceutical grade sterilizer. In some embodiments, the sterilizer is a commercial grade sterilizer.

Accordingly, also disclosed herein is a sterilized human milk product comprising: a bacterial aerobic plate count of less than 10 CFUs per gram of the sterilized human milk product; and a concentration of lactoferrin between 0.81 grams per liter to 13.28 grams per liter of the sterilized human milk product. In some embodiments, the sterilized human milk product further comprises a concentration of lysozyme between 0.012 grams per liter to 0.105 grams per liter of the sterilized human milk product. In some embodiments, the sterilized human milk product further comprises a concentration of lactalbumin between 1.87 grams per liter to 2.68 grams per liter of the sterilized human milk product. In some embodiments, the sterilized human milk product further comprises a concentration of anti-trypsin between 0.057 grams per liter to 0.40 grams per liter of the sterilized human milk product. In some embodiments, the sterilized human milk product further comprises a concentration of human milk oligosaccharides between 8.8 grams per liter and 20.0 grams per liter of the sterilized human milk product. In some embodiments, the sterilized human milk product further comprises a concentration of 2'fucosyllactose between 0.72 grams per liter and 4.3 grams per liter of the sterilized human milk product.

In some embodiments, the sterilized human milk product has, per 100 milliliters: (a) 63 to 67 calories, (b) 30 to 34 calories from fat, (c) 1.2 to 1.8 grams of saturated fat, (d) 10 to 16 mg of cholesterol, (e) 10 to 20 mg of sodium, (f) 4 to 10 grams of total carbohydrate, (g) 4 to 7 grams of sugars, (h) 1.2 to 1.6 grams of protein (i) 160 to 200 International Units of vitamin A, and (j) 27 to 35 mg of calcium. In some embodiments, the sterilized human milk product has, per 100 milliliters, greater than 63 calories. For example, the sterilized human milk product may have 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 calories per 100 milliliters of the sterilized human milk product.

In some embodiments, the sterilized human milk product has a reduced amount of *Bacillus Cereus* as compared to the amount of *Bacillus Cereus* in a raw human breast milk sample that the sterilized human milk product was sterilized from, wherein the reduced amount is at least a 500 log reduction of *Bacillus Cereus.* In some embodiments, the sterilized human milk product has a reduced amount of *Clostridium Botulinum* as compared to the amount of *Clostridium Botulinum* in the raw human breast milk sample that the sterilized human milk product was sterilized from, wherein the reduced amount is at least a 12 log reduction of *Clostridium Botulinum.*

In some embodiments, the sterilized human milk product is included in an aseptically packaged product. In some embodiments, the aseptically packaged product is packaged in one of a bottle, booster cup, paper carton, paper brick, or a pouch.

In some embodiments, the sterilized human milk product is included in an extended shelf life packaged product. In some embodiments, the extended shelf life packaged product is packaged in one of a bottle, booster cup, paper carton, paper brick, or a pouch.

Various objects, features, aspects and advantages of the present invention will become more apparent from the following detailed description of the embodiments of the invention, along with the accompanying drawings in which like numerals represent like components.

### 5. BRIEF DESCRIPTION OF THE DRAWINGS

The accompanying drawing(s), which are incorporated in and constitute a part of this specification, illustrate several aspects described below.
FIG. 1A depicts a flow process within a system for processing raw human breast milk to generate a sterilized human milk product, in accordance with an embodiment of the present invention.
FIG. 1B depicts devices of an optional bacterial clarifier (synonymously, milk clarifier) of the system, in accordance with an embodiment of the present invention.
FIG. 1C depicts devices of an optional milk standardizer of the system, in accordance with an embodiment of the present invention.
FIG. 2 depicts a system diagram of the milk sterilizer within the closed in-line sterilization system, in accordance with an embodiment of the present invention.
FIG. 3 depicts a graph of the milk product temperature while flowing through the milk sterilizer, in accordance with an embodiment of the present invention.
FIG. 4 depicts a flow chart for generating a sterilized human milk product, in accordance with an embodiment of the present invention.
FIG. 5 depicts a flow chart for sterilizing the milk product, in accordance with an embodiment of the present invention.
FIG. 6A tabulates parameters from multiple pilot-scale runs of the sterilization process described further in Example 7.1, in accordance with embodiments of the present invention.
FIG. 6B presents comparative nutritional data of a raw human milk sample and sterilized human milk product prepared in accordance with an embodiment of the present invention.
FIG. 6C presents compositional characteristics of a first batch of a raw milk sample and the sterilized human milk products resulting from sterilization Run 4 and Run 11 as defined in FIG. 6A, with the retention of each compositional characteristic calculated relative to the first batch of raw milk product.
FIG. 6D presents compositional characteristics of a second batch of a raw milk sample and the sterilized human milk products resulting from sterilization Run 7 and Run 14 as defined in FIG. 6A, with the retention of each compositional characteristic calculated relative to the second batch of raw milk product.
FIG. 6E presents quantified bacterial, mold and yeast content for raw human milk and sterilized human milk product.
FIGS. 7A and 7B present amounts of various supplements added per liter of milk sample to obtain fortified milk samples.
FIG. 7C depicts the processing parameters used to sterilize each fortified milk sample and *B. Cereus* log reduction of each fortified milk sample following sterilization.
FIG. 8A depicts analytical data of the composition of sterilized human milk product processed in Example 7.3.
FIG. 8B depicts microbial counts from Bottles 158, 1888, and 3151 in comparison to raw human milk.
FIG. 9A presents the analytical data and identifies the method reference used to determine various corresponding compositional components of the commercial-scale, sterilized and fortified human milk product produced in Example 7.4.
FIG. 9B depicts microbial counts of the aseptically bottled sterilized fortified human milk product in comparison to raw fortified human milk.

The figures use like reference numerals to identify like elements. A letter after a reference numeral, such as "135A," indicates that the text refers specifically to the element having that particular reference numeral. A reference numeral in the text without a following letter, such as "135," refers to any or all of the elements in the figures bearing that reference numeral (e.g. "bacterial clarifier 135" in the text refers to reference numerals "bacterial clarifier 135A" and/or "bacterial clarifier 135B" in the figures).

### 6. DETAILED DESCRIPTION

### 6.1. Definitions

It is to be understood that this invention is not limited to the particular embodiments described, as such may, of course, vary. It is also to be understood that the terminology used herein is for the purpose of describing particular embodiments only, and is not intended to be limiting, since the scope of this invention will be limited only by the appended claims.

The detailed description of the invention is divided into various sections only for the reader's convenience, and disclosure found in any section may be combined with that in another section. Unless defined otherwise, all technical and scientific terms used herein have the same meaning as commonly understood by one of ordinary skill in the art to which this invention belongs.

Throughout this disclosure, various aspects of the invention can be presented in a range format. Ranges include the recited endpoints. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Unless specifically stated or apparent from context, as used herein the term "**or**" is understood to be inclusive.

Unless specifically stated or apparent from context, as used herein, the terms "**a**", "**an**", and "**the**" are understood to be singular or plural. That is, the articles "**a**" and "**an**" are used herein to refer to one or to more than one *(i.e.,* to at least one) of the grammatical object of the article. By way of example, "an element" means one element or more than one element.

In this disclosure, "**comprises**," "**comprising**," "**containing**," "**having**," "**includes**," "**including**," and linguistic variants thereof have the meaning ascribed to them in U.S. Patent law, permitting the presence of additional components beyond those explicitly recited.

Unless specifically stated or otherwise apparent from context, as used herein the term "**about**" or "**approximately**" is understood as within a range of normal tolerance in the art, for example within 2 standard deviations of the mean and is meant to encompass variations of ±20% or ±10%, more preferably ±5%, even more preferably ±1%, and still more preferably ±0.1% from the stated value.

The term "**raw human milk**" or "**raw human breast milk**" composition is understood to mean human milk obtained from one or more female humans without any form of pasteurization, sterilization or decontamination having taken place. The raw composition may have been obtained from one or multiple donors, optionally pooled and optionally contaminated with *B. Cereus* and other bacterial, viral, mold, spores, or yeast pathogens.

The term "**sterilized human milk product**" composition is understood to mean the milk composition obtained after application of the method of sterilization in accordance with various embodiments of the present invention.

### 6.2. Systems and Methods for Generating a Sterilized Human Milk Product

### 6.2.1. Devices of the Sterilization System

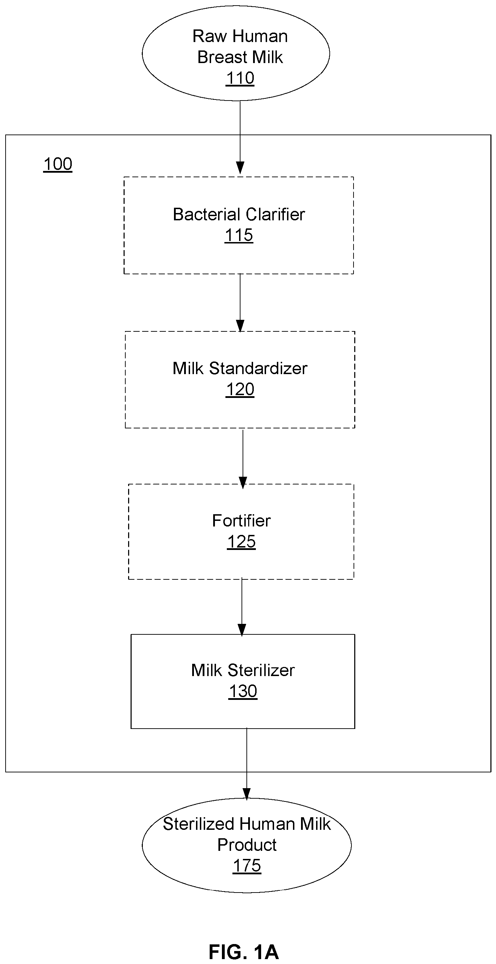

FIG. 1A depicts a flow process for processing raw human breast milk to generate a sterilized human milk product, in accordance with an embodiment of the present invention.

As depicted in FIG. 1A, raw human breast milk 110 is provided to a closed in-line sterilization system 100 that produces the sterilized human milk product 175. In various embodiments, once the raw human breast milk 110 enters the closed in-line sterilization system 100, the milk is not exposed to the external environment again until it is recovered as a sterilized human milk product 175.

The system comprises milk sterilizer 130.

In various embodiments, the closed in-line sterilization system 100 further includes one or more additional devices, such as those shown in FIG. 1A, such as bacterial clarifier (synonymously, milk clarifier) 115, milk standardizer 120, fortifier 125, or additional devices not shown in FIG. 1A, such as a milk homogenizer that homogenizes a milk sample or a deaerator that removes oxygen from a milk sample.

As an example, in some embodiments, such as those preferred for producing an engineered milk product suitable for remote consumption by premature infants, system 100 includes bacterial clarifier 115 and milk standardizer 120. In certain of these embodiments, system 100 further includes fortifier 125 and/or a milk homogenizer. In other embodiments, such as those preferred for producing an engineered milk product designed for adult consumption, system 100 includes fortifier 125 but does not include milk standardizer 120. In certain of these latter embodiments, system 100 further includes bacterial clarifier (milk clarifier) 115. In some embodiments, the devices in the closed in-line sterilization system 100 may be ordered differently. For example, the position of the bacterial clarifier (milk clarifier) 115, milk standardizer 120, and fortifier 125 within the system 100 may be interchanged.

In various embodiments, the devices of the closed in-line sterilization system 100 are in fluid communication with one another such that a continuous flow processing of the raw human breast milk 110 can occur as it is processed through system 100. As depicted in FIG. 1A, for example, the bacterial clarifier (milk clarifier) 115 may be in fluidic communication with the milk standardizer 120, which is in further fluid communication with the fortifier 125, which is further in fluid communication with the milk sterilizer 130. The tubes, pipes, or the like may be connected to the respective devices of the system 100 through welds, threads, or other appropriate connections. In various embodiments, the closed in-line sterilization system 100 includes one inlet (e.g., where the raw human breast milk 110 is inputted) and one outlet (e.g., where the sterilized human milk product 175 is outputted).

### 6.2.1.1. Raw human breast milk

The system 100 receives a batch of raw human breast milk 110.

In typical embodiments, the batch of raw human breast milk 110 is prepared by pooling a plurality of individual milk donations. In some embodiments, the plurality of milk donations is from a single donor. In typical embodiments, the plurality of milk donations includes milk from a plurality of donors.

In typical embodiments, each donor collects a plurality of raw human milk samples, freezing each sample as soon as fully expressed. The plurality of frozen raw human milk samples is then shipped frozen to be pooled and provided to the system 100 for processing.

Following receipt, the raw human breast milk from each donor is validated, that is, determined to meet certain requirements. In typical embodiments, one or more of an organoleptic test, a clot on boiling test, an alcohol test, an acidity test, a resazurin test, a drug test, and a milk inhibitor test is performed. Typically, raw milk donations that do not pass the validation test are discarded to prevent the donation from contaminating or adulterating a pooled raw human breast milk 110 batch that is to be provided to the system 100.

In various embodiments, prior to being provided to the closed in-line system 100, the raw human breast milk 110 is sampled to determine characteristics or concentration of components of the raw human breast milk 110. As used hereafter, characteristics of raw human breast milk 110 (and other milk products described below) include the density of the milk and total calories of the milk. Additionally, components of raw human breast milk 110 (and other milk products described below) include lipid/fat, protein (whey, casein, albumin, etc.), vitamins, cholesterol, ionic salts (e.g., sodium, calcium, iron, etc.) carbohydrates, immunoglobulins, and oligosaccharides. These initial characteristics and/or concentration of components of the raw human breast milk 110 may be provided to the milk combiner 150 for engineering the standardized human milk product. The process of engineering the standardized human milk product is described in further detail below.

In one embodiment, the total volume of a raw human breast milk 110 batch is 1,200 liters. In other embodiments, the total volume of a raw human breast milk 110 batch is more or less than 1,200 liters.

### 6.2.1.2. Bacterial clarifier (milk clarifier)

The bacterial clarifier (also, synonymously, milk clarifier) 115 is a device or apparatus capable of removing a fraction or all of pathogenic material and/or pathogens (e.g., bacterial, mold, spores, and or yeast contaminants) in the raw human breast milk 110. In one embodiment of the present system, the bacterial clarifier 115 is the first device of the system 100 that receives the raw human breast milk 110 and outputs clarified milk to the next device which, in some embodiments, is the milk standardizer 120. In other embodiments, bacterial clarifier 115 outputs clarified milk to fortifier 125. In other embodiments, bacterial clarifier 115 outputs clarified milk directly to milk sterilizer 130.

In some embodiments, bacterial clarifier 115 includes a single bacterial clarifier device 135 which outputs clarified milk 122 for input into the next device in the system 100. In some embodiments, the single bacterial clarifier removes up to 90% of pathogens, such as *B. Cereus* spores and/or *C*. *Botulinum* spores, from the milk.

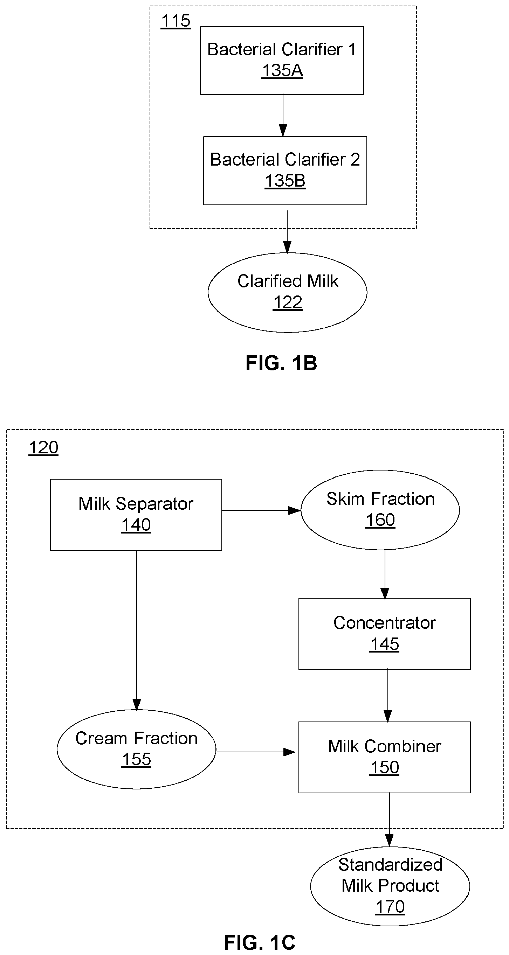

In some embodiments, bacterial clarifier 115 includes a plurality of bacterial clarifier devices. Reference is now made to FIG. 1B, which depicts two individual bacterial clarifiers 135A and 135B connected in series in accordance with an embodiment of the present invention. In this embodiment, the first bacterial clarifier 135A removes a first fraction of the pathogens and/or pathogenic material. The milk is then flowed to the second bacterial clarifier 135B which further removes a second fraction of pathogens and/or pathogenic material. In various embodiments, flowing the raw human breast milk 110 through first bacterial clarifier 135A and second bacterial clarifier 135B effects a double bacterial clarification process that removes up to 90% of pathogens from the milk. In some embodiments, the double bacterial clarification process removes up to 90% of *B. Cereus* spores from the milk. In some embodiments, the double bacterial clarification process removes up to 90% of *C*. *Botulinum* spores form the milk. The result of the double bacterial clarification process in these embodiments is clarified milk 122, which is provided to the next device in the system 100.

In other embodiments, the bacterial clarifier 115 employs more than two bacterial clarifiers 135 in series to further increase the amount of pathogenic clarification.

In various embodiments, each bacterial clarifier 135 is a centrifugal filtration device. An example of a suitable bacterial clarifier 135 is the GEA bacterial separator such as the GEA Pathfinder. In such embodiments, human breast milk that is provided to the bacterial clarifier 135 is centrifuged at a pre-determined speed for a duration of time. As the human breast milk 110 is centrifuged, solids above a particular density are collected according to the centrifugation speed and duration of centrifugation. These solids include larger pathogenic microbes (e.g., bacteria, viruses) as well as donor cells and other cellular material. The solids can then be further discharged at periodic intervals from the bacterial clarifier 135. The clarified human breast milk can then be provided to the next device in the system 100.

### 6.2.1.3. Milk standardizer

Returning to FIG. 1A, a milk standardizer 120 engineers a standardized human milk product to possess a target amount of components (e.g., specific nutrients) and/or target characteristics. Milk standardizer 120 thus ensures that the milk provided for optional fortification and for sterilization is standardized, such that each sterilized human milk product 175 batch that is generated has reduced variability in comparison to another sterilized human milk product 175 batch. Use of milk standardizer 120 is preferred for sterile milk products engineered for remote consumption by infants, and in particular, premature infants.

FIG. 1C depicts devices of a milk standardizer 120 of the system 100, in accordance with an embodiment of the present invention. For example, the milk standardizer 120 includes a milk separator 140, a concentrator 145, and a milk combiner 150 that generates the standardized milk product 170 that is provided to the next device of the system 100. A milk separator 140 separates a clarified milk sample into a cream fraction 155 and a skim fraction 160. The cream fraction 155 refers to the portion of the milk sample that includes a mixture of lipids (e.g., fats) whereas the skim fraction 160 includes components such as ionic salts, proteins (e.g., lactose, whey, casein micelles, immunoglobulins, albumin, and the like), water soluble vitamins, and human milk oligosaccharides (HMOs). In various embodiments, the milk separator 140 is capable of holding the temperature of the clarified milk, cream fraction 155, and skim fraction 160 at a particular temperature. For example, the milk separator 140 may apply refrigeration in order to hold the temperature of the clarified milk and each fraction at around 4°C - 20° C. In other embodiments, the milk separator 140 may apply heating in order to hold the temperature of the clarified milk and each fraction at around 45-65°C. Depending on the temperature at which the milk is to be separated, the milk separator 140 may be designed accordingly given that the viscosity of the clarified milk can vary substantially at different temperatures. For example, milk separators 125 may include discs that are responsible for separating the cream fraction 155 from the skim fraction 160. As such, a cold milk separator can be designed with fewer discs (more space between discs) in comparison to a hot milk separator in order to ensure that higher viscosity of milk at cold temperatures does not plug the milk separator 140.

In various embodiments, the milk separator 140 is a high speed centrifuge (otherwise known as an ultracentrifuge) that is capable of applying centrifugation speeds of 50,000 x g and higher. An example of a milk separator 140 is a Tetra Pak^{®} Separator. Given that the cream fraction 155 possesses a lower density than that of the skim fraction 160, the application of a pre-determined centrifugation speed for a duration of time effectively separates the skim fraction 160 from the cream fraction 155. As such, each individual fraction can be individually collected and subsequently processed. As depicted in FIG. 1C, the skim fraction 160 is provided to the concentrator 145 whereas the cream fraction 155 is provided directly to the milk combiner 150. In an embodiment, the cream fraction 155 is temporarily stored or held while the skim fraction 160 undergoes further processing.

The concentrator 145 further concentrates the skim fraction 160 into a retentate. In an embodiment, the concentrator 145 is a membrane filtration device that performs reverse osmosis on the skim fraction 160 in order to concentrate the components (e.g., salts, proteins, HMOs) that are in the skim fraction 160.

In some embodiments, the concentrator 145 concentrates the skim fraction 160 to obtain a retentate that has a target concentration of a component. For example, the concentrator 145 may detect, through a sensor or a similar device, the initial concentration of a component in the skim fraction 160. An example component concentration may be the protein concentration, individual amino acid concentrations, or vitamin concentrations. The retentate with a target concentration of a component is then provided to the milk combiner 150.

The milk combiner 150 engineers a standardized human milk product by combining portions of the cream fraction 155 and the retentate (e.g., concentrated skim fraction 160). In various embodiments, to achieve the desired concentrations in the standardized human milk product, the milk combiner 150 may further supplement water that was originally removed by the concentrator 145 when concentrating the skim sample 160.

In some embodiments, the milk combiner 150 is configured with one or more sensors for detecting characteristics of the cream fraction 155, retentate, or both. For example, a detectable characteristic may be a solution density or total calories of the cream fraction 155 or retentate. Alternatively or in addition, the sensor may be configured to detect concentrations of components in the cream fraction 155 or retentate. More specifically, a sensor of the milk combiner 150 may detect a concentration of protein in the retentate and a concentration of lipid (e.g., fat) in the cream fraction 155. Other components detectable by the sensor include specific amino acids, vitamins, carbohydrates, oligosaccharides, and immunoglobulins.

In various embodiments, the milk combiner 150 may be further configured to perform computations, or be in communication with a computing system. In some embodiments, the milk combiner 150 includes the computing system. Such a computing system is hereafter referred to as the computing system of the milk combiner 150. The computing system can calculate the desired portion of the cream fraction 155 and retentate that are to be combined according to the detected characteristics or concentration of components of the cream fraction 155 and retentate detected by the sensor of the milk combiner 150. Additionally, the computing system may be configured with a memory that, in some embodiments, stores a target characteristic or concentration of a component of the standardized human milk product.

As described above, one example of a stored target characteristic or target concentration of a component is an initial characteristic or initial concentration of a component of the raw human breast milk 110 that was previously sampled. As another example, a stored target characteristic or target concentration of a component may be fixed number that is pre-determined. Therefore, the milk combiner 150 attempts to engineer a standardized milk product that achieves the target characteristic or target concentration of a component. In various embodiments, achieving a target characteristic or concentration of component refers to achieving a characteristic or concentration of a component that is less than a percentage difference in comparison to the initial characteristic or initial concentration of a component of the raw human breast milk 110. In other embodiments, achieving a target characteristic or concentration of component refers to achieving a characteristic or concentration of a component that is less than a percentage difference in comparison to the fixed, pre-determined number.

As a more specific example, achieving a target density means that the standardized human milk product possesses a density that is less than a 10% difference in comparison to the stored target density. As another example, achieving a target protein concentration means that the standardized human milk product possesses a protein concentration that is less than a 5% difference in comparison to a stored target protein concentration. As a third example, achieving a target lipid concentration means that the standardized human milk product possesses a lipid concentration that is less than a 5% difference in comparison to a stored target lipid concentration.

In an example embodiment, the milk combiner 150 receives the cream fraction 155 and retentate and detects, using the one or more sensors, the characteristics and/or concentrations of components in the cream fraction 155 and retentate. The computing system of the milk combiner 150 compares the detected characteristics and/or concentrations of components to the stored target characteristics and/or concentration of components. In one embodiment, the computing system identifies a single characteristic or component as the standard and determines the portion of the cream fraction 155 and the retentate that are to be combined such that the standardized human milk product achieves the desired standard characteristic or component concentration. For example, the standardized human milk product can be engineered by combining a portion of the cream fraction 155 and retentate to possess a target concentration of protein in the standardized milk product.

In various embodiments, to engineer a standardized human milk product, the computing system prioritizes the characteristics and/or components of the standardized human milk product that are to be achieved. In one embodiment, the priority order of characteristics is as follows: 1) density, 2) protein concentration, and 3) lipid concentration. In other embodiments, other priority orders are established. As an example of this priority order, the computing system can first determine a first portion of the cream fraction 155 and first portion of the retentate that are to be combined to achieve the desired density (e.g., within a pre-designated percentage difference). Next, the computing system determines whether combining the first portion of the cream fraction 155 and the first portion of the retentate also achieves the desired protein concentration (e.g., within the percentage difference). If not, the computing system may adjust the volumes of the first portion of the cream fraction 155 and first portion of the retentate to satisfy the desired protein concentration while also maintaining the desired density. Similarly, the computing system can perform the same check for the lipid concentration. Therefore, in some embodiments, the computing system may calculate a standardized human milk product that achieves all characteristics that are included in the priority order of characteristics. In other embodiments, the computing system may generate a standardized human milk product that meets a subset of the characteristics according to the priority order of characteristics.

The milk combiner 150 combines the portions of the cream fraction 155 and the retentate, as calculated by the computing system of the milk combiner 150. In various embodiments, the milk combiner 150 further hydrates the combined portions of the cream fraction 155 and retentate. As such, the milk combiner 150 engineers a standardized human milk product 170. The standardized human milk product 170 is provided to the next device in the system 100.

In some embodiments, the milk standardizer 120 outputs the standardized human milk product to fortifier 125, as shown in FIG. 1A. The fortifier 125 can provide supplemental nutrients to the standardized human milk product as described below.

In some embodiments, the milk standardizer 120 provides the standardized milk product to a milk homogenizer that is located in-line between the milk combiner 150 and the milk sterilizer 130 (not shown in FIG. 1A).

In other embodiments, the milk combiner 150 provides the standardized milk product directly to the milk sterilizer 130 for further sterilization. Specifically, in these embodiments, the standardized milk product provided to the milk sterilizer 130 is not previously homogenized by any prior device in the closed in-line sterilization system 100. The lack of a homogenization step may help preserve the integrity of the components (e.g., proteins, HMOs, lipids, and the like) in the standardized milk product that may be otherwise disrupted or damaged by homogenization.

### 6.2.1.4. Milk homogenizer

If included in system 100, a milk homogenizer homogenizes the human milk product input thereto.

For example, the milk homogenizer forces, using a pressure-driven flow, the standardized human milk product through a small physical passage at a high velocity, thereby disrupting the fat globules of the standardized milk product. Homogenization of the standardized human milk product may improve the long term stability of the milk product, improve the flavor of the milk product, and/or improve the appearance of the milk product.

Depending on the intended use of the sterilized human milk product, these advantages may be outweighed by loss of nutritional value. In sterilized human milk product 175 intended for remote consumption by premature infants, homogenization by a milk homogenizer is currently not preferred.

### 6.2.1.5. Fortifier

In various embodiments, fortifier 125 provides supplemental nutrients to achieve a target concentration of the added supplemental nutrients.

In various embodiments, the fortifier 125 provides one or more of fat, antibodies (e.g., IgA, IgG, IgE, and the like), colostrum, such as bovine colostrum, protein (amino acids, whey, casein, albumin, etc.), vitamins, cholesterol, ionic salts (e.g., sodium, calcium, iron, etc.) carbohydrates, and human milk oligosaccharides as supplemental nutrients to the standardized human milk product. In some embodiments, fortifier 125 adds a high net nitrogen utilization (NNU) protein composition, as described in U.S. provisional application no. 62/439,408, filed December 27, 2016, incorporated herein by reference in its entirety.

### 6.2.1.6. Milk Sterilizer

The milk sterilizer 130 eradicates pathogens that are present in milk that is input thereto in order to ensure that the sterilized human milk product 175 is suitable for remote consumption by an individual, such as an adult, an adolescent, infants, and in particular, premature infants.

As discussed above, in some embodiments milk sterilizer 130 receives raw human breast milk 110 directly as input. In other embodiments, milk sterilizer 130 receives clarified human breast milk 122 as input. In other embodiments, milk sterilizer 130 receives standardized milk product 170, either prior clarified or not prior clarified, as input. In some embodiments, the milk that is input into milk sterilizer 130 has been fortified; in other embodiments, the milk that is input into milk sterilizer 130 has not been fortified. In some embodiments, the input milk is not homogenized. In other embodiments, the input milk is homogenized.

In preferred embodiments, the milk sterilizer 130 performs an ultra-high temperature sterilization process to generate the sterilized human milk product 175.

For example, the milk sterilizer 130 rapidly heats the human milk product to a target temperature. Additionally, the human milk product is held at the target temperature for a particular amount of time, hereafter referred to as a hold time. The target temperature, rate of heating, and hold time applied to the human milk product are selected to achieve the reduction of pathogenic content while maximizing the integrity of the nutritional components within the human milk product.

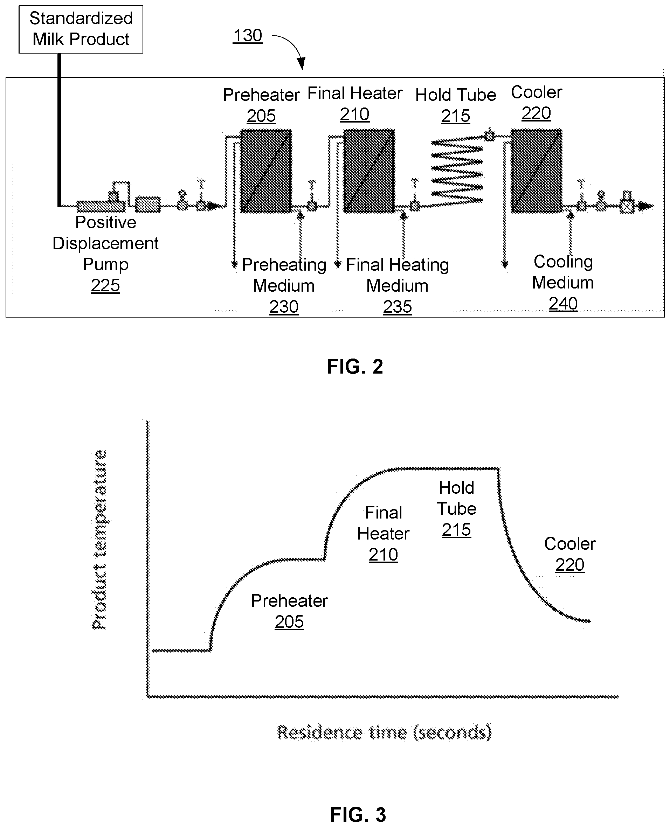

Reference is made to FIG. 2, which depicts a system diagram of the milk sterilizer 130 within the closed in-line sterilization system 100, in accordance with an embodiment of the present invention. In the depicted embodiment, the milk that is input into sterilizer 130 is a standardized milk product that is output by milk standardizer 120, optionally fortified by fortifier 125.

The milk sterilizer 130 may include a preheater 205, a final heater 210, a hold tube 215, and a cooler 220. Additionally, the milk sterilizer 130 may include a thermocouple in between each device in the milk sterilizer 130 such that the temperature of the milk flowing through the milk sterilizer 130 can be determined and/or monitored at each step of the sterilization process. As shown in FIG. 2, the milk sterilizer 130 may further include a positive displacement pump 225 that drives the input milk product through the subsequent devices of the milk sterilizer 130. Various embodiments of the milk sterilizer 130 may include fewer devices than those disclosed here in FIG. 2. As an example, the milk sterilizer 130 may include a single heater, as opposed to both a preheater 205 and a final heater 210, that performs a single heating process to heat the input milk product to a target temperature. Various embodiments of the milk sterilizer 130 may also include devices additional to those disclosed here in FIG. 2. For example, the milk sterilizer 130 may include a homogenizer located between any two of the devices depicted in FIG. 2.

In various embodiments, the milk sterilizer 130 meets good manufacturing practice (GMP) standards. For example, the tubing and connectors that come in contact with the input milk product meet GMP standards. In other embodiments, the milk sterilizer 130 is a pharmaceutical grade device that meets pharmaceutical grade standards, such that the sterilization process performed by the milk sterilizer 130 is a pharmaceutical grade sterilization process.

The milk sterilizer 130 is, in preferred embodiments, part of a closed in-line process that receives, as input, the milk product and provides, as output, the sterilized human milk product. The sterilization process performed by the milk sterilizer 130 can be a continuous flow process. More specifically, the milk flowing through the milk sterilizer 130 can flow at a single, constant flow velocity. In a series of embodiments, the milk flow velocity is between 0.25 gallons to 25 gallons per minute. In various embodiments, the milk flow velocity is between 0.25 gallons to 15 gallons per minute. In various embodiments, the milk flow velocity is between 0.25 gallons to 5 gallons per minute. In some embodiments, the milk flow velocity is between 5-100 gallons per minute.

The preheater 205 preheats the input milk product to a first target temperature. Referring now to FIG. 3, a graph is shown of the milk product temperature while flowing through the milk sterilizer 130, in accordance with an embodiment of the present invention. Specifically, the preheater 205 may preheat the input milk product from an initial temperature (e.g., between 0-25°C) to a first target temperature of 90°C. In other examples, the target temperature may be between 80°C and 100°C.

The preheater 205 may be a container, such as a vat, that is configured to receive the input milk product through a milk inlet. The preheater 205 outputs the preheated milk product through a milk outlet. Additionally, the preheater 205 can be configured to receive a preheating medium 230 through a heating inlet and outputs the preheating medium 230 through a heating outlet. In various embodiments, each of the milk inlet, milk outlet, heating inlet, and heating outlet are located on the preheater 205 such that countercurrent heat exchange can occur between an input milk product that flows through the milk inlet and milk outlet and a preheating medium that flows through the heating inlet and heating outlet. For example, the milk inlet and the heating outlet are on one side of the preheater 205. The milk inlet and the heating outlet can be adjacent to one another. Additionally, the milk outlet and the heating inlet are on an opposite side of the preheater 205. Here, the milk outlet and heating inlet may also be adjacent to one another. Therefore, the flow of the input milk product and the flow of the preheating medium 230 within the preheater 205 can be directionally opposite to each other, thereby enabling an efficient countercurrent heat exchange between the input milk product and the preheating medium 230 within the preheater 205.

In various embodiments, the preheating medium 230 fed into the preheater 205 through the heating inlet is at or above the first target temperature. Namely, the preheating medium 230 may be heated to or above the first target temperature in a separate chamber of the milk sterilizer 130 using electrical heating means. In other embodiments, the preheating medium 230 can be heated to or above the first target temperature in a separate chamber that resides within the preheater 205. Once the preheating medium 230 is at or above the first target temperature, it is then flowed through the preheater 205 to heat the input milk product through countercurrent heat exchange.

The preheating medium 230 may be any type of heating fluid with a high specific heat capacity such as water, mineral oils, as well as synthetic or organic based solutions. In some embodiments, the preheating medium 230 may include heated steam. In other embodiments, the preheating medium 230 may be held at a target pressure that ensures that the preheating medium 230 is in liquid form (e.g., liquid water) as opposed to being in a gas form (e.g., steam). This ensures that the countercurrent heat exchange occurs between a liquid preheating medium 230 and the liquid milk within the preheater 205.

The milk inlet and the milk outlet are connected within the preheater 205 by a tube through which the standardized milk flows. In some embodiments, the tube is a linear tube. In various embodiments, the tube may be a spiral tube. The spiral tube within the preheater 205 increases the surface area of the tube that is available for heat exchange between the preheating medium 230 and the input milk product within the spiral tube.

The tube is further configured to ensure that the input milk product is heated to the first target temperature. For example, the length of the tube may be selected such that the input milk product is preheated to the first target temperature and is able to equilibrate within a threshold range of the first target temperature while still in the spiral tube. As another example, the number of spirals of a spiral tube is chosen such that the input milk product is preheated to the first target temperature and is able to equilibrate within a threshold range of the first target temperature while still in the spiral tube. Additionally, the diameter of the tube may be between 0.25 inches and 10 inches. In some embodiments, the diameter of the tube is between 0.25 inches and 5 inches. In some embodiments, the diameter of the tube is between 0.25 inches and 1 inch. In some embodiments, the diameter of the tube is between 0.25 inches and 0.5 inches. In some embodiments, the diameter of the tube is 0.25 inches, 0.5 inches, 0.75 inches, 1 inch, 2 inches, 3 inches, 4 inches, 5 inches, 6 inches, 7 inches, 8 inches, 9 inches, or 10 inches.

Referring now to the final heater 210 of the milk sterilizer 130, in various embodiments, the final heater 210 may be similarly configured in comparison to the preheater 205. Generally, the description above regarding the preheater 205, unless explicitly stated otherwise here, may also be applied to the final heater 210.

For example, the final heater 210 can include a milk inlet that receives the preheated milk product from the preheater 205 and outputs the final heated milk product through a milk outlet. Similarly, the final heater 210 includes a heating inlet that receives the final heating medium 235 and a heating outlet that outputs the final heating medium 235. Each of the milk inlet, milk outlet, heating inlet, and heating outlet are located on the final heater 210 such that countercurrent heat exchange can occur between a preheated milk product that flows through the milk inlet and milk outlet and a final heating medium 235 that flows through the heating inlet and heating outlet. The milk inlet and the heating outlet may be on one side of the final heater 210, whereas the milk outlet and the heating inlet may be on an opposite side of the final heater 210. Therefore, countercurrent heat exchange can occur within the final heater 210 between the final heating medium 235 and the preheated milk product.

The final heater 210 differs from the preheater 205 in that the final heater 210 raises the temperature of the preheated milk product (e.g., at the first target temperature between 80°C and 100°C) to a second target temperature. Referring again to FIG. 3, the second target temperature may be between 130° C and 150° C. In one embodiment, the second target temperature may be between 138°C and 142°C. In one particular embodiment, the second target temperature is between 140°C and 141°C. To heat the preheated milk product to the second target temperature, the final heater 210 receives a final heating medium 235 that was previously heated to or above the second target temperature in a separate chamber. In various embodiments, the final heating medium 235 may be held under a target pressure higher than atmospheric pressure to ensure that the final heating medium 235 is in liquid form when the countercurrent heat exchange occurs between the preheated milk product and the final heating medium 235. The pressure that the final heating medium 235 is held under can be higher than the pressure that the preheating medium 230 is held under given that the temperature of the final heating medium 235 is higher.

The hold tube 215 is an insulated tube that holds the temperature of the heated milk product at or near the second target temperature for a hold time (e.g., a pre-determined amount of time). The pre-determined amount of time is selected such that the bacteria (e.g., *B. Cereus* and *C*. *Botulinum*) levels are reduced while maintaining the integrity of nutritional components (e.g. proteins, fats, immunoglobulins, oligosaccharides) in the sterilized milk product. In one embodiment, the hold time is up to 50 seconds. In another embodiment, the hold time is between 2 seconds and 20 seconds. In some embodiments, the hold time is between 3 seconds and 15 seconds. In some embodiments, the hold time is between 6 and 14 seconds. In some embodiments, the hold time is between 8 and 13 seconds. In one embodiment, the hold time is between 12 and 13 seconds.

To achieve a desired hold time, the hold tube 215 can be specifically configured. As depicted in FIG. 2, the hold tube 215 may be a spiral tube. Given a constant flow velocity of the milk through the tube, the spiral tube can be designed with additional or fewer spirals (e.g., additional or reduced length) to increase or decrease the hold time. Alternatively, for a constant length of the tube, the flow velocity of the milk through the tube can be tailored by adjusting the diameter of the hold tube 215. Therefore, increasing or decreasing the tube diameter results in a corresponding increase or decrease in the hold time. The milk sterilizer 130 can have one or more swappable hold tubes 215 that can be placed in between the final heater 210 and cooler 220 to achieve a variety of different hold times.

The cooler 220 receives and cools the heated milk product from the hold tube 215 to a target temperature. In some embodiments, the target temperature after cooling is between 15°C and 25°C. In one embodiment, the target temperature is room temperature (e.g., 23° C). In other embodiments, the target temperature is between 1° C and 8° C such as refrigeration temperature (e.g., 4° C).

In various embodiments, the cooler 220 can employ countercurrent heat exchange, similar to that of the preheater 205 and the final heater 210, in order to cool the heated milk product. Generally, the description above regarding the configuration of the preheater 205, unless explicitly stated otherwise here, may also be applied to cooler 220. Namely, the cooler 220 may have a milk inlet, milk outlet, cooling medium inlet, and cooling medium outlet. The inlets/outlets may be positioned such that countercurrent heat exchange between the heated milk product and the cooling medium 240 occurs. Similar to the preheater 205 and the final heater 210, the milk inlet and the cooling medium outlet may be on one side of the cooler 220, whereas the milk outlet and the cooling medium inlet are on an opposite side to enable countercurrent heat exchange. In other embodiments, the cooler 220 can employ other cooling methods to cool the heated milk product to the target temperature. The cooled milk product outputted by the cooling medium 240 is hereafter referred to as the sterilized human milk product 175.

### 6.2.1.7. Further processing and packaging

The sterilized human milk product 175 obtained from the closed in-line sterilization system 100 can be further processed and/or packaged.

As an example, the sterilized human milk product 175 may further undergo a packaging step. In various embodiments, this packaging step is an aseptic packing process that ensures that the sterilized human milk product 175 remains unadulterated and is safe for consumption. Such an aseptic packaging process may include the steps of package unscrambling, aseptic filling of the package with the sterilized human milk product 175, heat sealing of the package, labeling and coding of the package, tamper-evident sealing of the package, and container bundling. Aseptic packaging is currently preferred for sterilized human milk product 175 intended for consumption by infants, such as premature infants. In other embodiments, the packaging process is an extended shelf-life (ESL) packaging process. In various embodiments, the sterilized human milk product 175 is packaged into a bottle or a booster cup that facilitates the feeding of the sterilized human milk product 175 to an infant, such as a premature. In some embodiments, the packaging may be a paper carton, paper brick (e.g., a TETRA PAK^{®} aseptic brick), or a pouch.

In some embodiments, including those preferred for sterilized human milk product 175 suitable for remote consumption by infants, particularly premature infants, the sterilized human milk product 175 is aseptically packaged and meets pharmaceutical sterilization standards. More specifically, even if the sterilized human milk product 175 is exposed to oxygen (e.g., the packaging is opened), the sterilized human milk product 175 remains sterile and safe for consumption as pathogenic microbes have been sufficiently eradicated during the sterilization process. In other embodiments, the sterilized human milk product 175 is ESL packaged and therefore, can remain safe for consumption for extended periods of time after packaging.

### 6.2.1.8. Device sterilization standards

Altogether, the devices of the system 100 are each designed to meet certain standards.

For example, in one embodiment, the bacterial clarifiers 135A and 135B, the milk separator 140, the concentrator 145, and the milk combiner 150 each meet Grade "A" pasteurized milk ordinance (PMO) standards. Milk sterilizer 130 can be designed to meet PMO standards. In some embodiments, milk sterilizer 130 is designed to meet even higher standards; namely, the milk sterilizer 130 can be designed to meet GMP standards or pharmaceutical grade standards. As an example, to meet GMP or pharmaceutical grade standards, hygienic welds are employed while threaded fittings are avoided in the milk sterilizer 130. In various embodiments, the closed in-line sterilization system 100 is a clean-in-place (CIP) system, meaning that the closed in-line system 100 need not be disassembled to be cleaned.

### 6.2.2. Methods of Generating Sterilized Human Milk Product

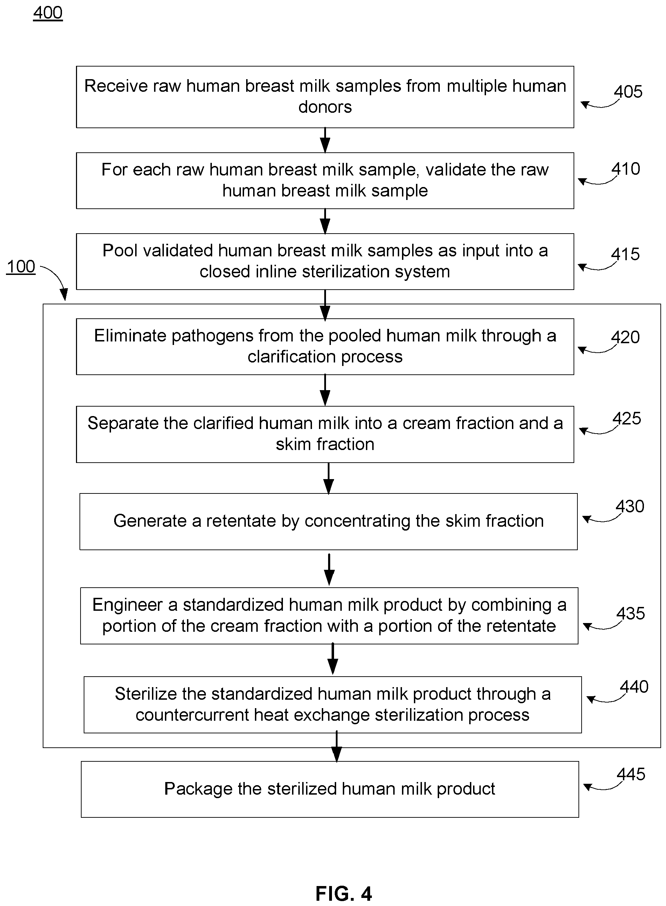

Reference is now made to FIG. 4, which depicts a flow chart for generating a sterilized human milk product 175, in accordance with an embodiment of the present invention.

The raw human breast milk 110 is first obtained. In typical embodiments, for example, individual raw breast milk donations (samples) are received 405 from multiple human donors. Each of the raw human breast milk donations is validated 410 to determine whether any donation fails to meet quality control standards. If a donation fails quality control standards, it is then discarded. The validated samples are pooled 415. The pooled validated samples represent the raw human breast milk 110 that is then provided as input into the system 100. In various embodiments, the system is a closed in-line sterilization system 100.

### 6.2.2.1. Methods of generating sterilized human milk product for remote consumption by infants

For sterilized human milk product intended for remote consumption by infants, such as premature infants, (i) maximal sterility and removal of pathogens, (ii) standardization, and (iii) retention of nutrients, is preferred.

Accordingly, with continued reference to FIG. 4, in preferred embodiments system 100 eliminates 420 a portion (e.g., up to 90%) of pathogens from the raw human breast milk 110 through a clarification process. In various embodiments, the process is a single bacterial clarification process performed by a single bacterial clarifier 135 of the system 100. In various embodiments, the process is a double bacterial clarification process performed by the bacterial clarifiers 135 of the system 100.

In typical embodiments intended for remote consumption by infants, such as premature infants, clarified milk 122 is then input into milk standardizer 120.

With reference to FIG. 1C, milk standardizer 120 then separates the clarified human milk into a cream fraction 155 and a skim fraction 160. In various embodiments, this separation process is performed by the milk separator 140 of the system 100.

The system 100 further generates a retentate by concentrating the skim fraction 160. In various embodiments, the concentrator 145 of the system 100 is a membrane filtration device that performs a reverse osmosis process on the skim fraction 160 in order to generate the retentate. The system 100 engineers 435 a standardized human milk product by combining a portion of the cream fraction 155 with a portion of the retentate. In various embodiments, combining portions of the cream fraction 155 and retentate is performed by the milk combiner 150 of the system 100. To determine the portions of the cream fraction 155 and the retentate that are to be combined, the milk combiner 150 detects characteristics or concentrations of components in each of the cream fraction 155 and the retentate. Therefore, a ratio of the cream fraction 155 and the retentate can be combined to achieve a target characteristic or concentration of components in the standardized human milk product.

For retention of nutrient value, it is currently preferred to omit homogenization.

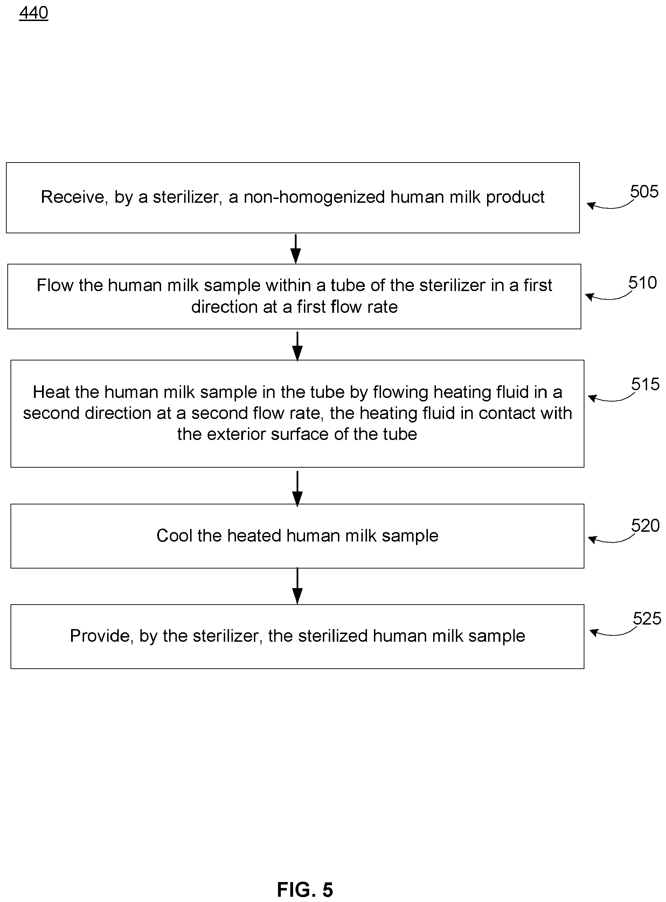

The system 100 sterilizes 440 the standardized human milk product. In various embodiments, the sterilization process is a countercurrent heat exchange process performed by the milk sterilizer 130 of the system 100. Reference is now made to FIG. 5, which depicts a flow chart of sterilizing the milk product, in accordance with an embodiment of the present invention. In particular, the flow process of FIG. 5 depicts the sterilization step 440 of FIG. 4 in further detail.

The milk sterilizer 130 of the system 100 may be a pharmaceutical grade device. The milk sterilizer 130 receives 505 the standardized human milk product which, in some embodiments, is non-homogenized. The milk sterilizer 130 performs the sterilization through a countercurrent heat exchange sterilization process. Namely, the standardized human milk product is flowed 510 through a tube of the milk sterilizer 130 in a first direction at a first flow rate. In various embodiments, the tube has a diameter between 0.25 to 10 inches. The standardized human milk product is flowed through the tube at a rate between 0.25 gallons/min and 25 gallons/min.

The milk sterilizer 130 heats 515 the standardized human milk product that is located within the tube by flowing a heating fluid in a second direction at a second flow rate. The heating fluid is in contact with the exterior surface of the tube. In various embodiments, the heating fluid is heated water and the second direction that the heated water is flowing is opposite of the first direction that the standardized human milk product is flowing. Thus, as the standardized human milk product flows through the tube, heat from the heated water flowing through the second tube can be readily transferred through the surface of the tube to heat the standardized human milk product.

In various embodiments, the milk sterilizer 130 preheats the standardized milk product and then further heats the preheated milk product to a target temperature, holding the milk product at the target temperature for a duration of time. The target temperature may be between 130°C and 150°C and the standardized human milk product held at the target temperature for 6-14 seconds. This process is refined to further eliminate pathogens that may reside in the standardized human milk product while also maintaining the components and nutrients in the standardized human milk product.

The milk sterilizer 130 cools 520 the heated human milk product down from the heated temperature to obtain the sterilized human milk product 175. In various embodiments, the human milk product is cooled to room temperature between 15°C to 25°C. In other embodiments, the human milk product is cooled to a refrigeration temperature between 1°C and 8°C.

The sterilized human milk product 175 is provided 525 by the milk sterilizer 130. For example, returning to the flow process of FIG. 4, the sterilized human milk product 175 is obtained from the milk sterilizer 130 and is packaged 445. In various embodiments, this may be an aseptic packaging process to ensure that the sterilized human milk product is not contaminated prior to being provided for feeding to an infant. In such embodiments, the aseptically packaged sterilized human milk product may be distributed without refrigeration.

In certain embodiments, the method further comprises fortifying the standardized milk product before sterilization.

In specific embodiments, the method further comprises fortifying the standardized milk product with colostrum. In typical embodiments, the colostrum is bovine colostrum. In certain embodiments, the colostrum is sheep or goat colostrum. In certain embodiments, the method comprises fortifying the standardized milk product with protein or a high net nitrogen utilization (NNU) protein composition, as described in U.S. provisional application no. 62/439,408, filed December 27, 2016, incorporated herein by reference in its entirety. In certain embodiments, the method comprises fortifying the standardized milk product with antibodies, vitamins, ionic salts (e.g., sodium, calcium, iron, etc.), fats, such as cholesterol, and/or carbohydrates, such as human milk oligosaccharides.

### 6.2.2.2. Methods of generating sterilized human milk product for remote consumption by adults

In certain embodiments of methods of generating sterilized human milk product for remote consumption by adults, system 100 eliminates 420 a portion (e.g., up to 90%) of pathogens from the raw human breast milk 110 through a clarification process. In various embodiments, the process is a single or double bacterial clarification process performed by one or more bacterial clarifiers 135 of the system 100. However, sterilized human milk product intended for remote consumption by adults requires less stringent sterility than sterilized human milk product intended for remote consumption by infants, and particularly premature infants. Accordingly, in some embodiments, the method omits bacterial clarification.

In some embodiments intended for remote consumption by adults, human breast milk, either with or without prior clarification, is then input into milk standardizer 120 and standardized as described above with respect to sterilized human milk product for remote consumption by infants. However, sterilized human milk product intended for remote consumption by adults requires less stringent standardization than sterilized human milk product intended for remote consumption by infants, and particularly premature infants. Accordingly, in some embodiments, the method omits standardization.

The sterilized human milk products intended for remote consumption by adults can usefully be fortified. In various embodiments, therefore, the method comprises fortifying the milk before sterilization by milk sterilizer 130.

In specific embodiments, the method further comprises fortifying the milk product with colostrum. In typical embodiments, the colostrum is bovine colostrum. In certain embodiments, the colostrum is sheep or goat colostrum.

In certain embodiments, the method comprises fortifying the milk product with protein. In particular embodiments, the method comprises fortifying the milk product with a high net nitrogen utilization (NNU) protein composition, as described in U.S. provisional application no. 62/439,408, filed December 27, 2016, incorporated herein by reference in its entirety. In the methods described herein, the high net nitrogen utilization (NNU) protein composition comprises free amino acids, that is, amino acids that are not bonded by peptide bond to any other amino acids.

In typical NNU fortification embodiments, at least 95% by weight of the amino acids in the high NNU composition are free amino acids. In certain embodiments, at least 96%, 97%, 98%, even at least 99% of the amino acids in the high NNU composition are free amino acids. In typical embodiments, less than 5% by weight of the amino acids in the high NNU composition are incorporated into peptides. In certain embodiments, less than 4%, 3%, 2%, even less than 1% by weight of the amino acids are incorporated into peptides. In particular embodiments, the composition contains no detectable peptides.

In typical embodiments, the high NNU composition comprises isoleucine, leucine, lysine, methionine, phenylalanine, threonine, tryptophan, and valine, each as the L-isomer. In some embodiments, the high NNU composition further comprises L-histidine. In some embodiments, the high NNU composition further comprises one or more non-essential amino acids.

In typical embodiments, the amino acids are present in the ratios described in U.S. Pat. No. 5,132,113, which is incorporated herein by reference in its entirety. In certain of these embodiments, the amino acids are present in the following proportions, in grams per 10 grams composition:
(a) from 1.217 to 1.647 isoleucine;
(b) from 1.827 to 2.735 leucine;
(c) from 1.260 to 2.359 lysine;
(d) from 0.232 to 0.778 methionine;
(e) from 0.843 to 1.314 phenylalanine;
(f) from 0.970 to 1.287 threonine;
(g) from 0.208 to 0.467 tryptophan; and
(h) from 1.260 to 1.900 valine.

In certain embodiments, the amino acids are present in the proportions set forth in one of the eight compositions (I-VIII) in Table 1, in grams per 10 grams composition:

| **Table 1.** | | | | | | | | |
|---|---|---|---|---|---|---|---|---|
| **Exemplary High NNU Protein Compositions** | | | | | | | | |
| | **I** | **II** | **III** | **IV** | **V** | **VI** | **VII** | **VIII** |
| isoleucine | 1.438 | 1.482 | 1.310 | 1.341 | 1.381 | 1.311 | 1.443 | 1.484 |
| leucine | 2.287 | 1.963 | 2.053 | 1.922 | 1.891 | 1.951 | 2.226 | 1.832 |
| lysine | 1.650 | 1.428 | 2.189 | 2.144 | 2.297 | 2.266 | 1.760 | 2.064 |
| methionine | 0.293 | 0.699 | 0.621 | 0.651 | 0.682 | 0.752 | 0.556 | 0.580 |
| phenylalanine | 0.943 | 1.288 | 1.029 | 1.027 | 1.029 | 0.959 | 1.100 | 1.067 |
| threonine | 1.226 | 1.111 | 1.107 | 1.211 | 1.113 | 1.119 | 1.041 | 1.136 |
| tryptophan | 0.448 | 0.368 | 0.293 | 0.338 | 0.318 | 0.256 | 0.317 | 0.371 |
| valine | 1.721 | 1.656 | 1.390 | 1.358 | 1.284 | 1.376 | 1.553 | 1.461 |

In typical embodiments, the high NNU composition is added in an amount sufficient to ensure a protein concentration of the final processed composition (F=6.38) of at least 1.0 g/100g (1.0 wt%), 1.1 wt%, 1.2 wt%, 1.3 wt%, or at least 1.4 wt%. In certain embodiments, the high NNU composition is added in an amount sufficient to ensure a protein concentration of the final processed composition of at least 1.30 wt%, 1.31 wt%, 1.32 wt%, 1.33 wt%, 1.34 wt%, 1.35 wt%, 1.36 wt%, 1.37 wt%, 1.38 wt%, 1.39 wt% or at least 1.40 wt%.

In some embodiments, the high NNU composition is added in an amount sufficient to ensure a protein concentration of the final processed composition of greater than 1.4 wt%. In certain of these high protein embodiments, the high NNU composition is added in an amount sufficient to ensure a protein concentration of the final processed composition of greater than 1.40 wt%, 1.41 wt%, 1.42 wt%, 1.43 wt%, 1.44 wt%, 1.45 wt%, 1.46 wt%, 1.47 wt%, 1.48 wt%, 1.49 wt%, even greater than 1.50 wt%. In certain of these high protein embodiments, the high NNU composition is added in an amount sufficient to ensure a protein concentration of the final processed composition of greater than 1.6 wt%, 1.7 wt%, 1.8 wt%, 1.9 wt%, or even greater than 2.0 wt%.

In various embodiments, the high NNU composition is added in an amount that provides at least 5% of the protein content of the final processed composition. In certain embodiments, the high NNU composition is added in an amount that provides at least 6%, 7%, 8%, 9%, or at least 10% of the protein content of the final processed composition. In particular embodiments, the high NNU composition is added in an amount that provides at least 15%, 20%, even at least 25% of the protein content of the final processed composition. In certain embodiments, the high NNU composition is added in an amount that provides at least 30%, 35%, 40%, 45% or even at least 50% of the protein content of the final processed composition.

In various embodiments, the high NNU composition is added in an amount that provides no more than 50% of the protein content of the final processed composition. In specific embodiments, the high NNU composition is added in an amount that provides no more than 45%, 40%, 35%, 30%, or 25% of the protein content of the final processed composition.

In a variety of embodiments, the high NNU composition is added in an amount that provides more than 50% of the protein content of the final milk product. These latter embodiments may be used alone, or in some embodiments will be mixed with unfortified milk before administration. In certain of these embodiments, the high NNU composition is added in an amount that provides at least 50%, 55%, 60%, 65%, 70%, 75% even at least 80%, 85%, 90% or 95% of the final process milk product.

In some embodiments, the milk product is fortified with flavorings. In some embodiments, the milk product is fortified with natural colorings.

In some embodiments, the milk product is fortified with vitamins. In specific embodiments, the milk product is fortified with vitamin C. In specific embodiments, the milk product is fortified with vitamin D.

In some embodiments, the milk product is fortified with minerals. In particular embodiments, the milk product is fortified with calcium. In certain embodiments, the milk product is fortified with magnesium.

In some embodiments, the milk product can be deaerated to remove gasses from the milk product. In various embodiments, therefore, the method comprises deaerating the milk product before sterilization by milk sterilizer 130. In some embodiments, the method comprises deaerating the milk product after fortifying the milk product.

In various embodiments of methods of producing sterilized human milk products intended for remote consumption by adults, homogenization is omitted. In other embodiments, the milk product is homogenized. In certain embodiments, the milk product is homogenized before sterilization. In other embodiments, the milk product is homogenized after sterilization.

### 6.3. Compositions of Sterilized Human Milk Product

In various embodiments, the sterilized human milk product 175 has 40-80 calories/100 mL, 50-70 calories/100 mL, or 60-70 calories/100 mL. In some embodiments, the sterilized human milk product 175 has 60, 61, 62, 63, 64, 65, 66, 67, 68, 69, or 70 calories/100 mL. In particular embodiments, the sterilized human milk product 175 has between 63.0 and 67.0 calories/100 mL. In certain embodiments, the sterilized human milk product 175 has 65.0 - 66.0 calories/100 mL.

In various embodiments, the sterilized human milk product 175 has 24-40 calories from fat per 100 milliliters (mL). In some embodiments, the sterilized human milk product 175 has 24-28 calories from fat/100 mL, 28-32 calories from fat/100 mL, 32-36 calories from fat/100 mL, or 36-40 calories from fat/100 mL. In some embodiments, the sterilized human milk product 175 has 28-36 calories from fat/100 mL. In some embodiments, the sterilized human milk product 175 has 28 calories from fat/100 mL, 29 calories from fat/100 mL, 30 calories from fat/100 mL, 31 calories from fat/100 mL, 32 calories from fat/100 mL, 33 calories from fat/100 mL, 34 calories from fat/100 mL, 35 calories from fat/100 mL, or 36 calories from fat/100 mL.

In various embodiments, the sterilized human milk product 175 has total fat content of at least 3.0 g/100 mL (3 wt%), 3.1 wt%, 3.2 wt%, 3.3 wt%, 3.4 wt%, 3.5 wt%, 3.6 wt%, 3.7 wt%, 3.8 wt%, 3.9 wt%, or 4.0 wt%. In some embodiments, the sterilized human milk product 175 has a total fat content of greater than 4.0 wt%.

In various embodiments, the sterilized human milk product 175 includes 1.2 milligrams (mg), 1.3 mg, 1.4 mg, 1.5 mg, 1.6 mg, 1.7 mg, or 1.8 mg of saturated fat per 100 mL of the sterilized human milk product 175. In various embodiments, the sterilized human milk product 175 includes 10 mg, 11 mg, 12 mg, 13 mg, 14 mg, 15 mg, or 16 mg of cholesterol per 100 mL of the sterilized human milk product 175. In various embodiments, the sterilized human milk product 175 includes 10 mg, 11mg, 12 mg, 13 mg, 14 mg, 15 mg, 16 mg, 17 mg, 18 mg, 19 mg, or 20 mg of sodium per 100 mL of the sterilized human milk product 175. In various embodiments, the sterilized human milk product 175 includes 4 g, 5 g, 6 g, 7 g, 8 g, 9 g, or 10 g of carbohydrates per 100 mL of the sterilized human milk product 175. In various embodiments, the sterilized human milk product 175 includes 4 g, 5 g, 6 g, or 7 g of sugar per 100 mL of the sterilized human milk product 175. In various embodiments, the sterilized human milk product 175 includes 1.2 g, 1.3 g, 1.4 g, 1.5 g, or 1.6 g of protein per 100 mL of the sterilized human milk product 175. In various embodiments, the sterilized human milk product 175 includes 160 International Units (IU), 170 IU, 180 IU, 190 IU, or 200 IU of Vitamin A per 100 mL of the sterilized human milk product 175. In various embodiments, the sterilized human milk product 175 includes 27 mg, 28 mg, 29 mg, 30 mg, 31 mg, 32 mg, 33 mg, 34 mg, or 35 mg per 100 mL of the sterilized human milk product 175.

In various embodiments, the sterilized human milk product 175 has a reduced amount of *Clostridium Botulinum* (*C. Botulinum*) as compared to the amount of C. *Botulinum* in the raw human breast milk 110 obtained from one or more human donors. In some embodiments, the reduction of C. *Botulinum* is between a 12-50 log reduction, a 20-45 log reduction, or a 30-42 log reduction. In some embodiments, the sterilized human milk product 175 has a 30, 31, 32, 33, 34, 35, 36, 37, 38, 39, 40, 41, or 42 log reduction of levels of *C*. *Botulinum* as compared to levels of *C*. *Botulinum* in raw human breast milk 110.

In various embodiments, the sterilized human milk product 175 has a reduced amount of *B. Cereus* as compared to the amount of *B. Cereus* in the raw human breast milk 110 obtained from one or more human donors. In various embodiments, the reduction of *B. Cereus* is greater than a 1000 log reduction of levels of *B. Cereus* as compared to levels of *B. Cereus* in raw human breast milk 110.

In various embodiments, the sterilized human milk product 175 has a bacterial aerobic plate count of less than 10 colony forming units (CFUs) per gram of the sterilized human milk product 175. In some embodiments, the sterilized human milk product 175 has yeast and mold counts that are below 10 CFUs per gram of the sterilized human milk product 175.

In various embodiments, the sterilized human milk product 175 retains above a certain percentage of components as compared to the raw human breast milk 110. In some embodiments, the percent retention of immunoglobulin A (IgA) is above 73%. In some embodiments, the percent retention of immunoglobulin M (IgM) is above 74%. In some embodiments, the percent retention of immunoglobulin G (IgG) is above 93%. In some embodiments, the percent retention of anti-trypsin is above 55%. In some embodiments, the percent retention of lactoferrin is above 74%. In some embodiments, the percent retention of lysozyme is above 64%. In some embodiments, the percent retention of lactalbumin is above 65%. In some embodiments, the percent retention of Alpha Casein is above 86%. In some embodiments, the percent retention of Beta Casein is above 89%. In some embodiments, the percent retention of Kappa Casein is above 81%. In some embodiments, the percent retention of osteopontin is above 80%. In some embodiments, the percent retention of total HMOs is above 79%. In some embodiments, the percentage retention of fucosylated HMOs is above 90%. Furthermore, in some embodiments, the percentage retention of 2'-fucosyllactose is above 90%. In some embodiments, the percentage retention of 3'-fucosyllactose is above 90%. In some embodiments, the percentage retention of sialylated HMOs is above 90%. In some embodiments, the percentage retention of non-fucosylated HMOs is above 89%.

Referring to absolute concentrations of the components in the sterilized human milk product 175, in some embodiments, the concentration of lactoferrin in the sterilized human milk product 175 is between 0.81 g/L and 13.28 g/L. In other embodiments, the concentration of lactoferrin in the sterilized human milk product 175 is between 1 g/L and 12 g/L. In some embodiments, the concentration of lactoferrin in the sterilized human milk product 175 is 2 g/L, 3 g/L, 4 g/L, 5 g/L, 6 g/L, 7 g/L, 8 g/L, 9 g/L, 10 g/L, or 11 g/L.

In some embodiments, the concentration of lysozyme in the sterilized human milk product 175 is between 0.012 g/L and 0.105 g/L. In some embodiments, the concentration of lysozyme in the sterilized human milk product 175 is between 0.015 g/L and 0.105 g/L. In some embodiments, the concentration of lysozyme in the sterilized human milk product 175 is 0.021 g/L, 0.022 g/L, 0.023 g/L, 0.024 g/L, 0.025 g/L, 0.026 g/L, 0.027 g/L, 0.028 g/L, 0.029 g/L, 0.030 g/L, 0.031 g/L, 0.032 g/L, 0.033 g/L, 0.034 g/L, 0.035 g/L, 0.036 g/L, 0.037 g/L, 0.038 g/L, 0.039 g/L, 0.040 g/L, 0.041 g/L, 0.042 g/L, 0.043 g/L, 0.044 g/L, 0.045 g/L, 0.046 g/L, 0.047 g/L, 0.048 g/L, 0.049 g/L, 0.050 g/L, 0.051 g/L, 0.052 g/L, 0.053 g/L, 0.054 g/L, 0.055 g/L, 0.056 g/L, 0.057 g/L, 0.058 g/L, 0.059 g/L, 0.060 g/L, 0.061 g/L, 0.062 g/L, 0.063 g/L, 0.064 g/L, 0.065 g/L, 0.066 g/L, 0.067 g/L, 0.068 g/L, 0.069 g/L, 0.070 g/L, 0.071 g/L, 0.072 g/L, 0.073 g/L, 0.074 g/L, 0.075 g/L, 0.076 g/L, 0.077 g/L, 0.078 g/L, 0.079 g/L, 0.080 g/L, 0.081 g/L, 0.082 g/L, 0.083 g/L, 0.084 g/L, 0.085 g/L, 0.086 g/L, 0.087 g/L, 0.088 g/L, 0.089 g/L, 0.090 g/L, 0.091 g/L, 0.092 g/L, 0.093 g/L, 0.094 g/L, 0.095 g/L, 0.096 g/L, 0.097 g/L, 0.098 g/L, 0.099 g/L, 0.100 g/L, 0.101 g/L, 0.102 g/L, 0.103 g/L, 0.104 g/L, or 0.105 g/L.

In some embodiments, the concentration of lactalbumin in the sterilized human milk product 175 is between 1.87 g/L to 2.68 g/L. In some embodiments, the concentration of lactalbumin in the sterilized human milk product 175 is between 2.0 g/L to 2.6 g/L. In some embodiments, the concentration of lactalbumin in the sterilized human milk product 175 is between 2.2 g/L to 2.4 g/L. In some embodiments, the concentration of lactalbumin in the sterilized human milk product 175 is 2.21 g/L, 2.22 g/L, 2.23 g/L, 2.24 g/L, 2.25 g/L, 2.26 g/L, 2.27 g/L, 2.28 g/L, 2.29 g/L, 2.30 g/L, 2.31 g/L, 2.32 g/L, 2.33 g/L, 2.34 g/L, 2.35 g/L, 2.36 g/L, 2.37 g/L, 2.38 g/L, or 2.39 g/L.

In some embodiments, the concentration of anti-trypsin in the sterilized human milk product 175 is between 0.057 g/L to 0.40 g/L. In some embodiments, the concentration of anti-trypsin in the sterilized human milk product 175 is between 0.10 g/L to 0.30 g/L. In some embodiments, the concentration of anti-trypsin in the sterilized human milk product 175 is 0.11 g/L, 0.12 g/L, 0.13 g/L, 0.14 g/L, 0.15 g/L, 0.16 g/L, 0.17 g/L, 0.18 g/L, 0.19 g/L, 0.20 g/L, 0.21 g/L, 0.22 g/L, 0.23 g/L, 0.214 g/L, 0.25 g/L, 0.26 g/L, 0.27 g/L, 0.28 g/L, or 0.29 g/L.

In some embodiments, the concentration of HMOs in the sterilized human milk product 175 is between 8.8 g/L to 20.0 g/L. In some embodiments, the concentration of HMOs in the sterilized human milk product 175 is between 10 g/L to 18 g/L. In some embodiments, the concentration of HMOs in the sterilized human milk product 175 is between 14 g/L to 16 g/L. In some embodiments, the concentration of HMOs in the sterilized human milk product 175 is 10 g/L, 11 g/L, 12 g/L, 13 g/L, 14 g/L, 15 g/L, 16 g/L, 17 g/L, or 18 g/L.

In some embodiments, the concentration of fucosylated HMOs in the sterilized human milk product 175 is between 5.46 g/L and 5.6 g/mL. In some embodiments, the concentration of fucosylated HMOs in the sterilized human milk product 175 is 5.47 g/L, 5.48 g/L, 5.49 g/L, 5.50 g/L, 5.51 g/L, 5.52 g/L, 5.53 g/L, 5.54 g/L, 5.55 g/L, 5.56 g/L, 5.57 g/L, 5.58 g/L, or 5.59 g/L.

In some embodiments, the concentration of 2'-fucosyllactose in the sterilized human milk product 175 is between 0.72 g/L and 4.3 g/L. In some embodiments, the concentration of 2'-fucosyllactose in the sterilized human milk product 175 is between 1 g/L and 4 g/L. In some embodiments, the concentration of 2'-fucosyllactose in the sterilized human milk product 175 is 1.1 g/L, 1.2 g/L, 1.3 g/L, 1.4 g/L, 1.5 g/L, 1.6 g/L, 1.7 g/L, 1.8 g/L, 1.9 g/L, 2.0 g/L, 2.1 g/L, 2.2 g/L, 2.3 g/L, 2.4 g/L, 2.5 g/L, 2.6 g/L, 2.7 g/L, 2.8 g/L, 2.9 g/L, 3.0 g/L, 3.1 g/L, 3.2 g/L, 3.3 g/L, 3.4 g/L, 3.5 g/L, 3.6 g/L, 3.7 g/L, 3.8 g/L, 3.9 g/L, or 4.0 g/L.

In some embodiments, the concentration of 3'-fucosyllactose in the sterilized human milk product 175 is between 0.92 g/L and 0.93 g/L. In some embodiments, the concentration of sialylated HMOs in the sterilized human milk product 175 is between 1.08 g/L and 1.11 g/L. In some embodiments, the concentration of sialylated HMOs in the sterilized human milk product 175 is between 1.09 g/L and 1.10 g/L. In some embodiments, the concentration of non-fucosylated HMOs in the sterilized human milk product 175 is between 2.46 and 2.56 g/L. In some embodiments, the concentration of non-fucosylated HMOs in the sterilized human milk product 175 is 2.47 g/L, 2.48 g/L, 2.49 g/L, 2.50 g/L, 2.51 g/L, 2.52 g/L, 2.53 g/L, or 2.54 g/L.

### 7. EXAMPLES

The following examples are put forth so as to provide those of ordinary skill in the art with a complete disclosure and description of how to make and use the subject invention, and are not intended to limit the scope of what is regarded as the invention. Efforts have been made to ensure accuracy with respect to the numbers used (e.g. amounts, temperature, concentrations, etc.) but some experimental errors and deviations should be allowed for. Unless otherwise indicated, parts are parts by weight, molecular weight is average molecular weight, temperature is in degrees centigrade; and pressure is at or near atmospheric.

### 7.1. Example 1: Pilot-scale ultra-high temperature sterilization of human milk

Fourteen different sterilization runs were performed on human milk obtained from human donors using an Ultra-High Temperature Lab-25 electric high viscosity hybrid unit (MicroThermics^{®}, Raleigh, NC) to determine whether ultra-high temperature sterilization could be applied to human milk to achieve high levels of sterilization with simultaneous preservation of desired components of the human milk.

The human milk was validated and pooled prior to sterilization.

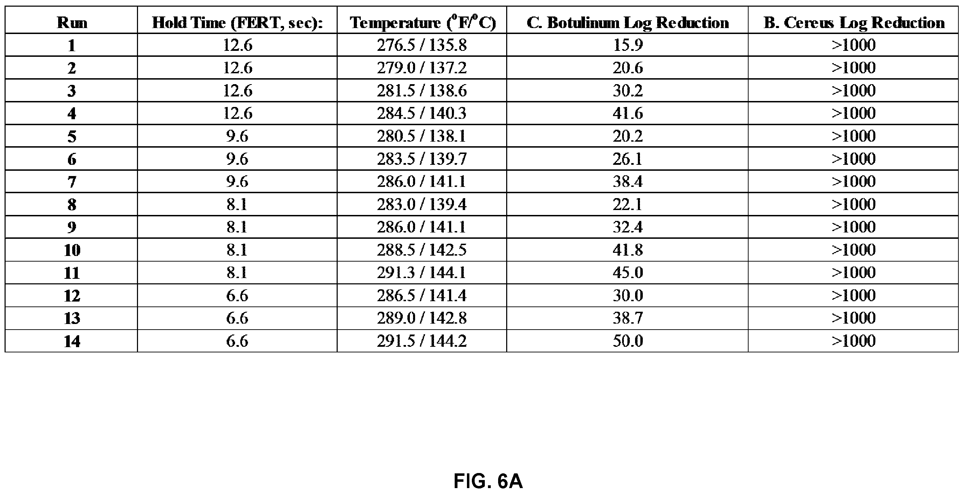

The human milk was heated to a target temperature while undergoing countercurrent heat exchange and held at the target temperature for a duration of time. FIG. 6A depicts the parameters (e.g., temperature and hold time) from various sterilization runs. Generally, hold times, recited in terms of fluid elements residence time (FERT), ranged from 6.6 seconds up to 12.6 seconds with a temperature range from 135°C to 145°C. The FERT refers to the residence time of elements (e.g., milk) flowing through the center of the hold tube. A flow rate of 0.25 gallons/min of the human milk product was held constant across all runs. Additionally depicted in FIG. 6A is the log reduction of *C*. *Botulinum* and *B. Cereus* for each run. Generally, the data confirm between a 12-50 log reduction in C. *Botulinum* content and greater than 1000 log reduction of *B. Cereus.* This meets and exceeds the standard 12 log reduction (12 D concept) for sterilization of a given food.

Of note, an additional human milk sample underwent ultra-high temperature sterilization using the processing parameters shown for Run 8 (e.g., hold time = 8.1 seconds, temperature = 286°F/141.1°C). Following sterilization, the additional sterilized human milk product also exhibited a greater than 1000 log reduction of *B. Cereus.*

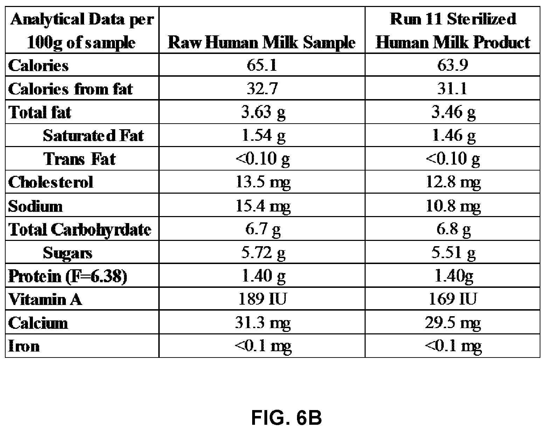

Reference is now made to FIG. 6B, which depicts comparative nutritional data of a raw human milk sample and sterilized human milk product from Run 11, which included a hold time of 8.1 seconds and a target temperature of 144.1°C. As shown in FIG. 6B, for a 100 g sample, minimal differences were observed in vital nutritional components and properties - including total calories, total fat, cholesterol, sodium, carbohydrates, protein, vitamin A, vitamin C, calcium, iron, amino acids, vitamins - as compared to raw human milk. Namely, the majority of components (except for sodium) and properties of the sterilized human milk product exhibited a difference of 6 percent or less in comparison to the components and characteristics of the raw human milk sample.

The additional sterilized human milk product that underwent ultra-high temperature sterilization using the processing parameters of Run 8 (e.g., hold time = 8.1 seconds, temperature = 286°F/141.1°C) was further analyzed for protein quality using the standardized Food and Agricultural Organization (FAO) method (e.g., FAO Paper 51 (1991)). The protein digestibility corrected amino acid score (PDCAAS) of this sterilized human milk product was 73.3 (w/w %).

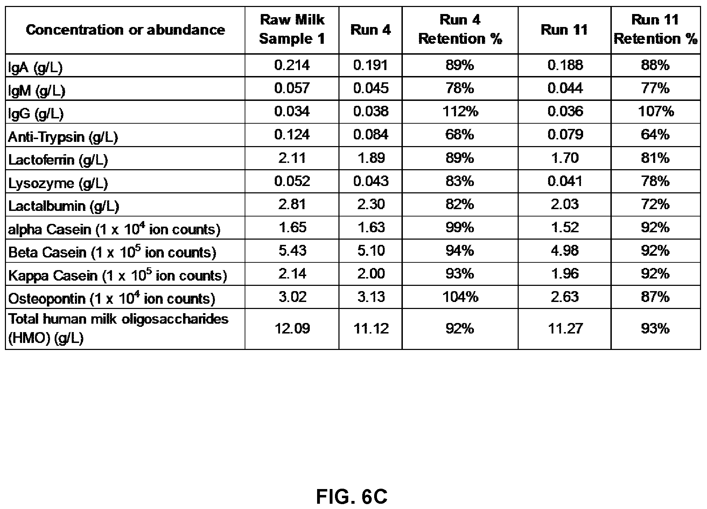

Reference is now made to FIG. 6C, which depicts comparative concentrations and abundance of components measured by mass spectrometry in a first batch of raw human breast milk in comparison to sterilized human milk product for two of the experimental runs: Run 4 and Run 11. Each of the sterilized human milk product obtained from Runs 4 and 11 was derived from the first batch of raw human breast milk. The components quantified by mass spectrometry include immunoglobulins (IgA, IgM, and IgG), proteins (Anti-Trypsin, Lactoferrin, Lysozyme, Lactalbumin, alpha casein, beta casein, kappa casein, and osteopontin), and human milk oligosaccharides (HMOs). Further depicted in FIG. 6C is the retention percentage of each of the components in the standardized human milk product for each respective sterilization run.

Specifically, the Run 4 sterilization process resulted in retention percentages of a majority of components that are above 80%, aside from IgM (78% retention) and Anti-Trypsin (68% retention). Importantly, FIG. 6C includes concentration and retention numbers of total HMOs, a component of human milk that is often overlooked in sterilized human milk products. Here, the sterilized human milk product generated using the Run 4 sterilization process retains 92% of total HMOs. The Run 11 sterilization process retained 93% of total HMOs.

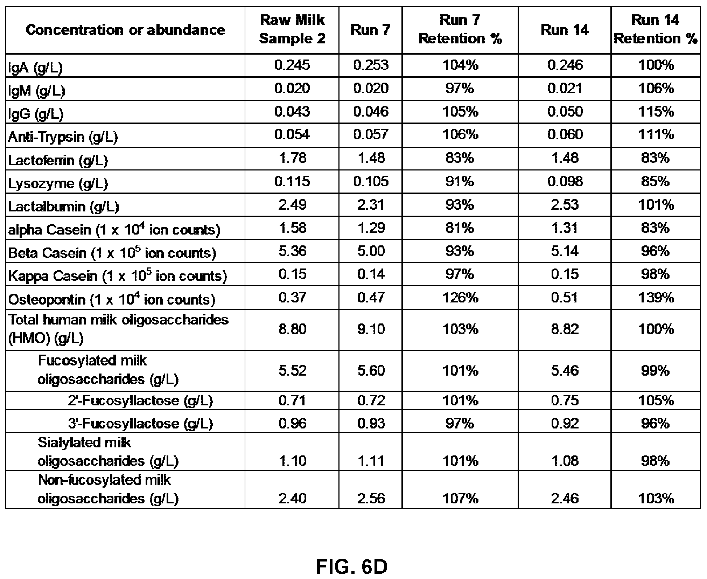

Reference is now made to FIG. 6D, which further depicts comparative concentrations and abundance of components measured by mass spectrometry in sterilized human milk products in comparison to a second raw milk batch. Specifically, FIG. 6D depicts sterilized milk products obtained as a result of Run 7 and Run 14, as shown in FIG. 6A. Each of the sterilized human milk product obtained from Runs 7 and 14 was derived from the second batch of raw human breast milk. Here, the measured components of the sterilized milk products include immunoglobulins (IgA, IgM, and IgG), proteins (Anti-Trypsin, Lactoferrin, Lysozyme, Lactalbumin, alpha casein, beta casein, kappa casein, and osteopontin), and human milk oligosaccharides (HMOs). Furthermore, the abundance of individual elements of the HMOs were further characterized, the individual elements including fucosylated HMOs, sialylated HMOs, and non-fucosylated HMOs. Additionally, the abundance of individual elements of fucosylated HMOs, including 2'-fucosyllactose (2-FL) and 3'-fucosyllactose (3-FL), were further quantified. Given that 2-FL and 3-FL have been implicated in protecting against infectious diseases and reducing inflammation, the retention of 2-FL and 3-FL through the sterilization process is of interest.

Here, the Run 7 and Run 14 sterilization processes each resulted in retention percentages of components above 80%, with the majority of components experiencing retention percentages above 90%. The retention percentages of some components are noted to exceed 100%, though these results may be a consequence of measurement error. Of note, the retention percentage of total HMOs for both Run 7 and Run 14 was near 100%. Similarly, the retention percentage of 2-FL and 3-FL for Run 7 and Run 14 were also each near 100%. This demonstrates that the sterilization process does not damage HMOs and, therefore, can ensure the bioavailability of important HMOs, such as 2-FL and 3-FL, that may have a therapeutic effect when consumed.

As shown in both FIG. 6C and FIG. 6D, the sterilization process achieves high retention percentages of components in the sterilized human milk product. Given that

FIG. 6C and 6D each recites a percentage retention for each component, the absolute concentration of each component in the sterilized human milk product will be dependent on the concentration of each component in the raw human breast milk when obtained from a donor. As shown by the first and second batches of raw human milk sample (e.g., FIG. 6C and 6D) and further confirmed by prior literature, the concentrations of various components (e.g., the components depicted in FIG. 6C and 6D) in raw human breast milk varies widely. For example, prior literature has shown that lactoferrin levels in breast milk differ as a function of time post-delivery (e.g., 1 day, 14 weeks, and 6 months). *See* Shashiraj, F. M. et al., European Journal of Clinical Nutrition 60:903-908 (2006). Additionally, levels of components in breast milk differs based on whether the birth was a preterm or full term birth. *See* Mehta, R. et al., Journal of Perinatology 31:58-62 (2011).

Specifically, the concentration of lactoferrin in raw human breast milk has been shown to range up to 14.92 g/L. *See* Turn, C.G. et al., Journal of Perinatology 37(5):507-512 (2017). Additionally, the concentration of lactoferrin in raw human breast milk has been shown to be as low as 1 g/L. *See* Montagne, P. et al., Advances in Experimental Medicine and Biology, vol 501, Springer, Boston, MA. Therefore, given the 81% retention achieved in Run 11 (as depicted in FIG. 6C) of the sterilization process, the concentration of lactoferrin in a sterilized milk product can range from 0.81 g/L up to 13.28 g/L. Additionally, the lysozyme concentration in raw human breast milk can be 0.015 g/L. *See* Hsu, Y. et al., Pediatrics and Neonatology 55:449-454 (2014). Therefore, given the 91% retention achieved in Run 7 of the sterilization process, the concentration of lysozyme in a sterilized milk product can range from 0.012 g/L to 0.105 g/L (Run 7 of FIG. 6D). Additionally, the lactalbumin concentration in raw human breast milk can range from 2.28 g/L up to 3.27 g/L. *See* Affolter, M. *et al*., *Nutrients* 8(8):504 (2016). Therefore, given the 82% retention achieved in Run 4 of the sterilization process, the concentration of lactalbumin in a sterilized milk product can range from 1.87 g/L to 2.68 g/L. Additionally, the anti-trypsin concentration in raw human breast milk can range from 0.1 g/L to 0.4 g/L. *See* Chowanadisai, W. et al., Am. J. Clin. Nutr. 76(4):828-833 (2002). Therefore, given the near 100% retention of anti-trypsin achieved in Run 7 and Run 11, the concentration of anti-trypsin in a sterilized milk product can range from 0.057 (Run 7) up to 0.4 g/L.

Additionally, the human milk oligosaccharide concentration in human breast milk can range up to 20 g/L. *See* Gabrielli, O. et al., Pediatrics, 128(6):e1520-31 (2011). FIG. 6C depicts a raw milk sample with 12 g/L of human milk oligosaccharides whereas FIG. 6D depicts a raw milk sample with 8.8 g/L of human milk oligosaccharides. Therefore, the concentration of HMOs in a sterilized milk product can range from 8.8 g/L (see Run 14) up to 20 g/L, given the near 100% retention observed in Run 7 and 14. Additionally, the 2'fucosyllactose concentration in human breast milk can range from 1.1 to 4.3 g/L. *See* Puccio, J. Pediatr. Gastroenterol. Nutr. 64(4): 624-631 (2017). Therefore, given the near 100% retention achieved in Run 7 and Run 14, the concentration of 2'fucosyllactose in a sterilized milk product can range from 0.72 g/L (Run 7) to 4.3 g/L.

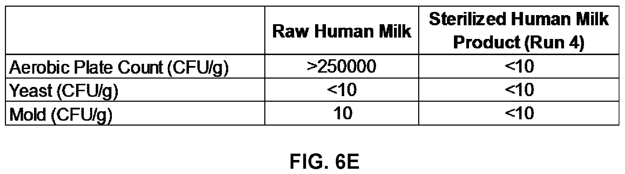

FIG. 6E depicts quantified bacterial, mold and yeast content for raw human milk and sterilized human milk product. Here, the sterilized human milk product corresponds to Run 4 of the sterilization process as described above. The raw human milk corresponds to the same sample prior to sterilization. A standard aerobic plate count of each sample was performed to determine the level of microorganisms in the sample. Serial dilutions of each sample were plated on an agar petri dish and incubated (48 hours at 37°C) to allow for microorganism colony formation. Colony forming units (CFUs) of each serial dilution were visualized and quantified to determine the aerobic plate count of each petri dish sample. Specifically, the sterilized human milk product demonstrated significantly lower bacterial counts (< 10 CFUs per gram) in comparison to the raw human milk (>250,000 CFUs per gram).

The systems and methods as disclosed herein provide a sterilized human milk product with a low bioburden of *B. Cereus* and *C. Botulinum* content. Thus, the sterilized human milk product is safe for consumption by premature infants. Additionally, the sterilized human milk product retains levels of nutritional components comparable to the raw human milk composition. As such, the sterilized human milk product is suitable for consumption by premature infants.

### 7.2. Example 2: Pilot-scale ultra-high temperature sterilization of fortified human milk

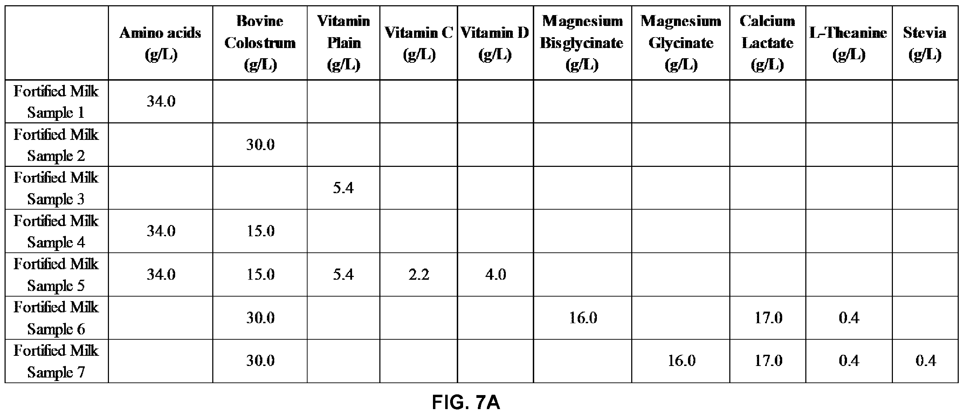

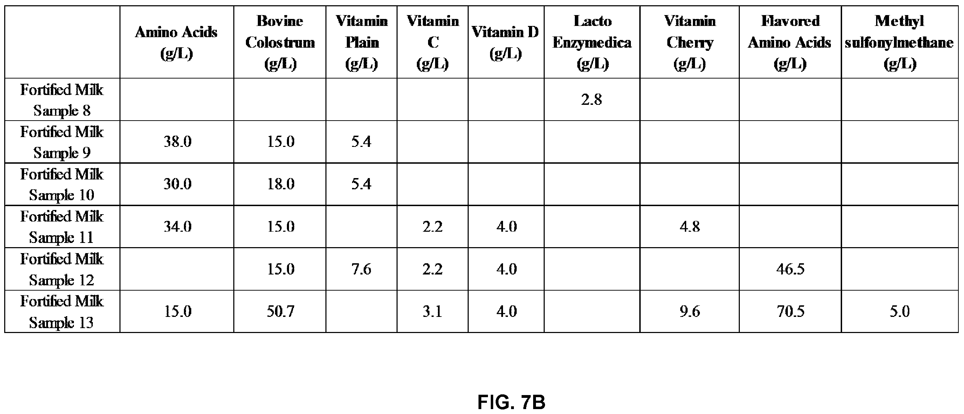

Human milk was obtained from human donors, validated, and pooled. The pooled human milk was divided into thirteen batches and each batch was fortified with one or more supplements. Supplements include amino acids, bovine colostrum, vitamin plain, vitamin C, vitamin D, magnesium bisglycinate, magnesium glycinate, calcium lactate, L-theanine, stevia, lacto enzymedica, vitamin cherry, flavored amino acids, and methylsulfonylmethane. Reference is made to FIG. 7A and 7B, which presents the formulation of each fortified milk sample. Specifically, FIG. 7A and 7B present the amount of each supplement, in grams, that was added to each liter of human milk to obtain each fortified milk sample.

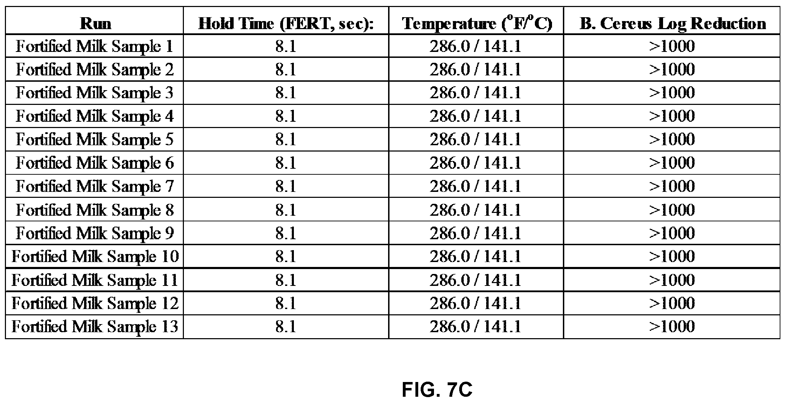

Each fortified milk sample underwent ultra-high temperature sterilization using an Ultra-High Temperature Lab-25 electric high viscosity hybrid unit (MicroThermics^{®}, Raleigh, NC). Reference is made to FIG. 7C, which presents the processing parameters used to sterilize each fortified milk sample. Specifically, each fortified human milk sample was heated to a target temperature of 286°F/141.1°C while undergoing countercurrent heat exchange and held at the target temperature for 8.1 seconds. A flow rate of 0.25 gallons/min of each fortified human milk product was held constant across all runs.

Additionally depicted in FIG. 7C is the log reduction of *B. Cereus* for each run. For each sterilized fortified milk sample, a greater than 1000 log reduction of *B. Cereus* was observed in comparison to the corresponding raw fortified milk sample counterpart.

### 7.3. Example 3: Commercial-scale ultra-high temperature sterilization of human milk

Ultra-high temperature sterilization of a commercial-scale batch of human milk was performed. A total of 50,000 fluid ounces (~1480 liters) of human milk from various human donors were validated and pooled prior to sterilization. The pooled 50,000 fluid ounces of human milk was sterilized using the Tetra Therm^{®} Aseptic Flex to provide an ultra-high temperature treatment at the Tetra Pak Pilot Plant (Denton, TX), which is an FDA-registered food production facility.

The 50,000 fluid ounces of human milk was heated to a target temperature of 289.4°F/143°C while undergoing countercurrent heat exchange and held at the target temperature for 10.3 seconds. The human milk was flowed through the sterilization system at a flow rate of 8 gallons/min. Following sterilization of the human milk, the sterilized milk product underwent in-line homogenization at 1800-2500 psi. The homogenized sterilized milk product was then aseptically bottled into 3 and 330 mL bottles.

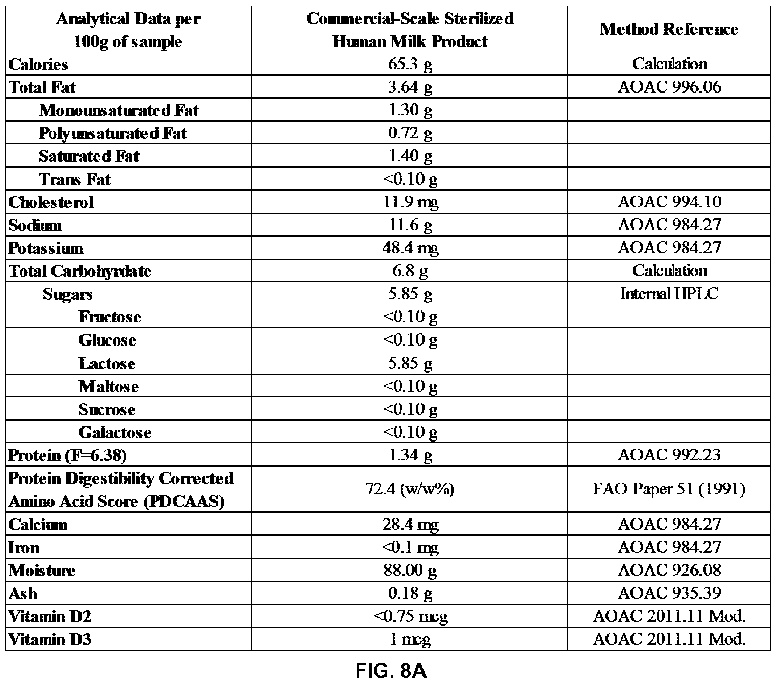

A selection of bottles was provided to Merieux Nutrisciences (Crete, IL) for determination of the composition of the sterilized human milk product. Specifically, the sampled sterilized human milk product was tested for the following characteristics and components: density (g/mL), calories (g), total fat (g), monounsaturated fat (g), polyunsaturated fat (g), saturated fat (g), trans fat (g), cholesterol (mg), sodium (g), potassium (mg), total carbohydrates (g), sugars (g), fructose (g), glucose (g), lactose (g), maltose (g), sucrose (g), galactose (g), protein (g), calcium (mg), iron (mg), moisture (g), ash (g), vitamin D2 (mcg) and vitamin D3 (mcg). Additionally, the sterilized human milk product was further tested for protein quality which is quantified using the protein digestibility corrected amino acid score (PDCAAS). FIG. 8A presents the analytical data. Additionally, FIG. 8A identifies the method reference used to determine various corresponding analytical components. Components were detected using official Association of Official Agricultural Chemists (AOAC) methods, Food and Agricultural Organization (FAO) methods, internal high performance liquid chromatography, or database calculations. The density of the sample of sterilized human milk product was 1.016 g/mL.

Of note, the protein digestibility corrected amino acid score (PDCAAS) of the sterilized milk product was 72.4 (w/w%). In comparison, a pasteurized human milk sample obtained from the Mothers Milk Bank in San Jose, CA, which had previously undergone conventional pasteurization treatment at 145°F/62.8°C for 30 minutes, had a PDCAAS of 64.9 (w/w%). Altogether, the sterilized milk product possesses a 10% improvement in protein quality in comparison to conventional pasteurized human milk samples.

Selected bottles were also provided to Merieux NutriSciences (Salida, CA) for the determination of *B. Cereus,* aerobic plate count, total microbial count, yeast, and mold in the aseptically bottled, sterilized human milk product. Bottles were selected from early during the sterilization run (bottle number 158), later during the sterilization run (bottle number 1888), and still later during the sterilization run (bottle number 3151).

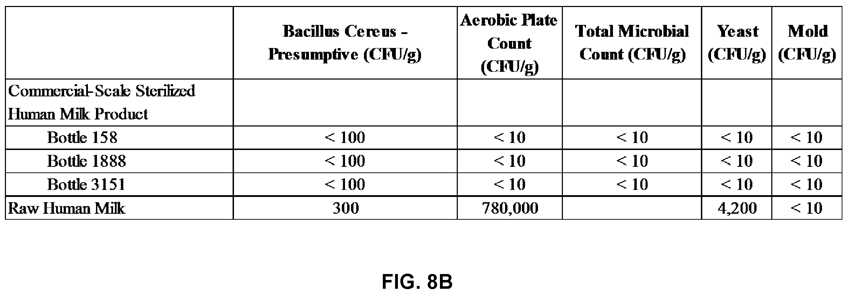

Reference is now made to FIG. 8B, which depicts microbial counts from Bottles 158, 1888, and 3151 in comparison to raw human milk. *B. Cereus* counts were determined using the standardized AOAC 980.31 method for determining *B. Cereus* in foods. Aerobic plate count of each sample was determined using the standardized AOAC 966.23 method. Total microbial count was determined using the standardized USP<61> test for total aerobic microbial count. Yeast and mold were determined using the standardized USP <61> test for Microbiological Examination of Non-sterile Products.

Each of the three aseptically bottled sterilized, homogenized, human milk products exhibited low levels (< 100 CFU/g) of presumptive *B. Cereus* in comparison to a raw human milk sample, which exhibited significantly higher levels (300 CFU/g) of presumptive *B. Cereus.* Furthermore, each of the three aseptically bottled sterilized, homogenized, human milk products exhibited a low (< 10 CFU/g) aerobic plate count and low total microbial count (<10 CFU/g). On the contrary, raw human milk sample exhibited a significantly higher (780,000 CFU/g) aerobic plate count. Each of the three aseptically bottled sterilized and homogenized human milk products further exhibited lower levels (< 10 CFU/g) of yeast in comparison to a raw human milk sample, which exhibited significantly higher levels (4,200 CFU/g) of yeast. The aseptically bottled sterilized and homogenized human milk product contained in bottles 158, 1888, and 3151 pass industry-standard Quality Control requirements and are considered Commercially Sterile.

### 7.4. Example 4: Commercial-scale ultra-high temperature sterilization of fortified human milk

A total of 27,000 fluid ounces (800 liters) of human milk from various human donors were validated, pooled, and fortified prior to ultra-high temperature sterilization. Specifically, the human milk was fortified with bovine colostrum (50.7 g per liter of human milk), amino acids (70.5 g per liter of human milk), ascorbic acid (3.1 g per liter of human milk), and vitamin D (4.0 g per liter of human milk). Organic stevia extract was also added. The 27,000 fluid ounces of fortified human milk was sterilized using the Tetra Therm^{®} Aseptic Flex to provide an ultra-high temperature treatment at the Tetra Pak Pilot Plant (Denton, TX), which is an FDA-registered food production facility.

The 27,000 fluid ounces of fortified human milk was heated to a target temperature of 289.4°F/143°C while undergoing countercurrent heat exchange and held at the target temperature for 10.3 seconds. The fortified human milk was flowed through the sterilization system at a flow rate of 8 gallons/min. Following sterilization of the fortified human milk, the sterilized fortified milk product underwent in-line homogenization at 1800-2500 psi. The sterilized fortified milk product was then obtained and aseptically bottled into 330 mL bottles.

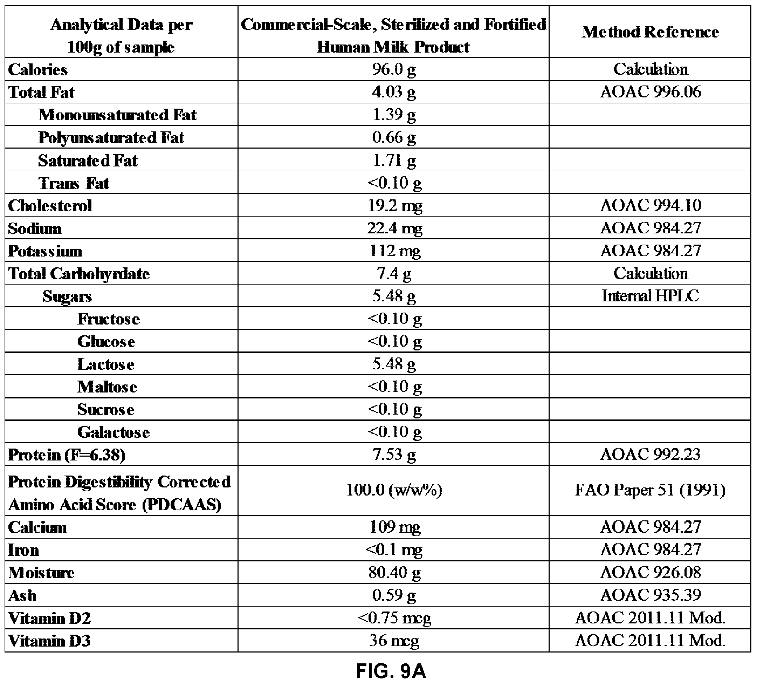

A bottle was provided to Merieux Nutrisciences located in Crete, IL for determination of the composition of the sterilized fortified human milk product. Specifically, the sterilized fortified human milk product was tested for the following characteristics and components: density (g/mL), calories (g), total fat (g), monounsaturated fat (g), polyunsaturated fat (g), saturated fat (g), trans fat (g), cholesterol (mg), sodium (g), potassium (mg), total carbohydrates (g), sugars (g), fructose (g), glucose (g), lactose (g), maltose (g), sucrose (g), galactose (g), protein (g), calcium (mg), iron (mg), moisture (g), ash (g), vitamin D2 (mcg) and vitamin D3 (mcg). Additionally, the sterilized fortified human milk product was further tested for protein quality using the PDCAAS. FIG. 9A presents the analytical data and identifies the method reference used to determine various corresponding analytical components. Components were detected using official AOAC methods, internal high performance liquid chromatography, or database calculations. The density of the sample of sterilized human milk product was 1.052 g/mL. Of note, the PDCAAS of the sterilized fortified milk product was 100.0 (w/w%), which indicates the high protein quality of the sterilized fortified milk product likely due to the fortification process.

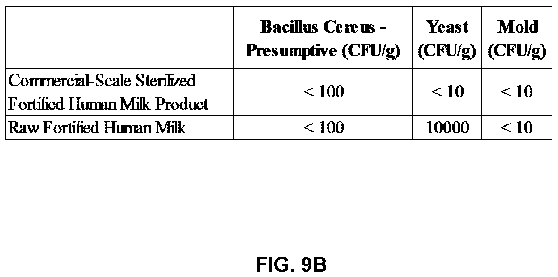

A bottle of the sterilized fortified human milk product was provided to Merieux NutriSciences located in Salida, CA for determining the presence of *B. Cereus,* yeast, and mold. Reference is now made to FIG. 9B, which depicts microbial counts of the aseptically bottled sterilized fortified human milk product in comparison to raw fortified human milk. *B. Cereus* counts were determined using the standardized AOAC 980.31 method for determining *B. Cereus* in foods. Yeast and mold were determined using the standardized USP <61> test for Microbiological Examination of Non-sterile Products.

The aseptically bottled sterilized fortified human milk product exhibited low levels (< 100 CFU/g) of presumptive *B. Cereus,* low levels (< 10 CFU/g) of yeast, and low levels (< 10 CFU/g) of mold. In particular, the level of yeast (< 10 CFU/g) in the aseptically bottled, sterilized fortified human milk product was significantly lower in comparison to the raw fortified human milk counterpart (10,000 CFU/g).

The present application and invention further includes the subject matter of the following numbered clauses:
1. A method of sterilizing human milk to produce a sterilized human milk product, the method comprising:
   receiving, by a sterilizer located within a closed in-line system, as an input, a human milk sample;
   flowing the human milk sample through a tube of the sterilizer in a first direction at a first flow rate;
   heating the human milk sample in the tube by flowing heating fluid in a second direction at a second flow rate, wherein the heating fluid is in contact with an exterior surface of the tube; and
   cooling, by the sterilizer, the heated human milk sample.
2. The method of clause 1, wherein heating the human milk sample in the tube comprises:
   raising a temperature of the human milk sample to within a temperature range of 130°
   C and 150° C; and
   holding the temperature of the human milk sample within the temperature range for a duration of 3 to 15 seconds.
3. The method of clause 1, wherein heating the human milk sample in the tube comprises:
   raising a temperature of the human milk sample to within a temperature range of 135°
   C and 145° C; and
   holding the temperature of the human milk sample within the temperature range for a duration of 6 to 14 seconds.
4. The method of clause 1, wherein heating the human milk sample in the tube comprises:
   raising a temperature of the human milk sample to within a temperature range of 138
   C and 142° C; and
   holding the temperature of the human milk sample within the temperature range for a duration of 8 to 13 seconds.
5. The method of clause 1, wherein heating the human milk sample in the tube comprises:
   raising a temperature of the human milk sample to within a temperature range of 140°
   C and 141° C; and
   holding the temperature of the human milk sample within the temperature range for a duration of 12 to 13 seconds.
6. The method of any one of clauses 1-5, wherein a length of the portion of the tube and the first flow rate of the human milk sample are each previously chosen to hold the temperature of the human milk sample within the temperature range for the duration.
7. The method of any one of clauses 1-6, wherein the first tube of the sterilizer has a diameter between 0.25 and 10 inches.
8. The method of any one of clauses 1-7, wherein the first flow rate of the human milk sample through the tube of the sterilizer is between 0.25 gallons per minute and 25 gallons per minute.
9. The method of any one of clauses 1-8, wherein the human milk sample received by the sterilizer is non-homogenized.
10. The method of any one of clauses 1-8, wherein the human milk sample is homogenized prior to being received by the sterilizer or homogenized after being cooled by the sterilizer.
11. The method of any one of clauses 1-10 further comprising the step, prior to the sterilizer receiving the human milk sample, of:
   eliminating pathogens from a raw human breast milk sample through one or more clarification processes.
12. The method of clause 11 further comprising, after the one or more clarification processes and prior to the sterilizer receiving the human milk sample, the steps of:
   separating a clarified human milk sample into a cream sample and a skim sample;
   generating a retentate by performing a concentration process on the skim sample; and
   engineering a standardized human milk sample to produce an engineered human milk product by combining a portion of the cream sample with a portion of the obtained retentate.
13. The method of clause 12, wherein combining a portion of the cream sample with a portion of the obtained retentate comprises:
   detecting an initial characteristic of the cream sample;
   comparing the detected initial characteristic of the cream sample to a target characteristic for the engineered human milk product; and
   determining the portion of the cream sample and the portion of the obtained retentate based on the comparison.
14. The method of any one of clauses 1-13 further comprising the step of, subsequent to cooling of the sterilized human milk product, packaging, through an aseptic process, the sterilized human milk product.
15. The method of any one of clauses 1-14, wherein the sterilizer is a pharmaceutical grade sterilizer.
16. The method of any one of clauses 1-15, wherein the sterilized human milk product has at least 63 calories per 100 milliliters of the sterilized human milk product.
17. The method of any one of clauses 1-16, wherein the sterilized human milk product has a total fat content of at least 3.0 g/100 mL (3 wt%).
18. The method of any one of clauses 1-17, wherein the sterilized human milk product retains above 88% of immunoglobulin A in comparison to a raw human breast milk sample that the sterilized human milk product derives from.
19. The method of any one of clauses 1-18, wherein the sterilized human milk product retains above 77% of immunoglobulin M in comparison to a raw human breast milk sample that the sterilized human milk product derives from.
20. The method of any one of clauses 1-19, wherein the sterilized human milk product retains above 93% of immunoglobulin G in comparison to a raw human breast milk sample that the sterilized human milk product derives from.
21. The method of any one of clauses 1-20, wherein the sterilized human milk product retains above 64% of anti-trypsin in comparison to a raw human breast milk sample that the sterilized human milk product derives from.
22. The method of any one of clauses 1-21, wherein the sterilized human milk product retains above 81% of lactoferrin in comparison to a raw human breast milk sample that the sterilized human milk product derives from.
23. The method of any one of clauses 1-22, wherein the sterilized human milk product retains above 78% of lysozyme in comparison to a raw human breast milk sample that the sterilized human milk product derives from.
24. The method of any one of clauses 1-23, wherein the sterilized human milk product retains above 72% of lactalbumin in comparison to a raw human breast milk sample that the sterilized human milk product derives from.
25. The method of any one of clauses 1-24, wherein the sterilized human milk product retains above 92% of Alpha Casein in comparison to a raw human breast milk sample that the sterilized human milk product derives from.
26. The method of any one of clauses 1-25, wherein the sterilized human milk product retains above 92% of Beta Casein in comparison to a raw human breast milk sample that the sterilized human milk product derives from.
27. The method of any one of clauses 1-26, wherein the sterilized human milk product retains above 92% of Kappa Casein in comparison to a raw human breast milk sample that the sterilized human milk product derives from.
28. The method of any one of clauses 1-27, wherein the sterilized human milk product retains above 87% of osteopontin in comparison to a raw human breast milk sample that the sterilized human milk product derives from.
29. The method of any one of clauses 1-28, wherein the sterilized human milk product retains above 92% of total human milk oligosaccharides in comparison to a raw human breast milk sample that the sterilized human milk product derives from.
30. The method of any one of clauses 1-29, wherein the sterilized human milk product retains above 90% of fucosylated human milk oligosaccharides in comparison to a raw human breast milk sample that the sterilized human milk product derives from.
31. The method of any one of clauses 1-30, wherein the sterilized human milk product retains above 90% of 2'-fucosyllactose in comparison to a raw human breast milk sample that the sterilized human milk product derives from.
32. The method of any one of clauses 1-31, wherein the sterilized human milk product retains above 90% of 3'-fucosyllactose in comparison to a raw human breast milk sample that the sterilized human milk product derives from.
33. The method of any one of clauses 1-32, wherein the sterilized human milk product retains above 90% of sialylated human milk oligosaccharides in comparison to a raw human breast milk sample that the sterilized human milk product derives from.
34. The method of any one of clauses 1-33, wherein the sterilized human milk product retains above 90% of non-fucosylated human milk oligosaccharides in comparison to a raw human breast milk sample that the sterilized human milk product derives from.
35. A closed, in-line processing system comprising:
   a sterilizer comprising:
   a tube configured to flow a human milk sample in a first direction at a first flow rate; and
   a container that a portion of the tube resides within, wherein the container is configured to flow a heating fluid in contact with an exterior surface of the portion of the tube in a second direction at a second flow rate to heat the human milk sample flowing through the portion of the first tube to a predetermined temperature for a duration.
36. The system of clause 35, wherein the predetermined temperature is within a temperature range of 130° C and 150° C and the predetermined duration is between 3 and 15 seconds.
37. The system of clause 35, wherein the predetermined temperature is within a temperature range of 135° C and 145° C and the predetermined duration is between 6 and 14 seconds.
38. The system of clause 35, wherein the predetermined temperature is within a temperature range of 138° C and 142° C and the predetermined duration is between 8 and 13 seconds.
39. The system of clause 35, wherein the predetermined temperature is within a temperature range of 140° C and 141° C and the predetermined duration is between 12 and 13 seconds.
40. The system of any one of clauses 35-39, wherein the first flow rate of the human milk sample through the tube of the sterilizer is between 0.25 gallons per minute and 25 gallons per minute.
41. The system of any one of clauses 35-40 further comprising:
   one or more bacterial clarifiers, each bacterial clarifier configured to eliminate pathogens from a raw human breast milk sample;
   a milk separator in fluid communication with the last in series of the at least one bacterial clarifier, the milk separator configured to separate a clarified human milk sample into a cream sample and a skim sample;
   a milk concentrator in fluid communication with the milk separator, the milk concentrator further configured to generate a retentate by performing a reverse osmosis process on the skim sample received from the milk separator; and
   a milk combiner configured to combine a portion of the cream sample from the milk separator and a portion of the retentate from the milk concentrator.
42. The system of clause 41, wherein the milk combiner is in fluid communication with the sterilizer, and wherein a homogenizer is not located in the closed, in-line processing system prior to the sterilizer.
43. The system of any one of clauses 35-42, wherein the system is capable of undergoing a clean-in-place (CIP) method of cleaning.
44. The system of any one of clauses 35-43, wherein the sterilizer is a pharmaceutical grade sterilizer.
45. The system of any one of clauses 35-43, wherein the sterilizer is a commercial grade sterilizer.
46. A sterilized human milk product comprising:
   a bacterial aerobic plate count of less than 10 CFUs per gram of the sterilized human milk product; and
   a concentration of lactoferrin between 0.81 grams per liter to 13.28 grams per liter of the sterilized human milk product.
47. The sterilized human milk product of clause 46, further comprising a concentration of lysozyme between 0.012 grams per liter to 0.105 grams per liter of the sterilized human milk product.
48. The sterilized human milk product of clause 46 or 47, further comprising a concentration of lactalbumin between 1.87 grams per liter to 2.68 grams per liter of the sterilized human milk product.
49. The sterilized human milk product of any one of clauses 46-48, further comprising a concentration of anti-trypsin between 0.057 grams per liter to 0.40 grams per liter of the sterilized human milk product.
50. The sterilized human milk product of any one of clauses 46-49, further comprising a concentration of human milk oligosaccharides between 8.8 grams per liter and 20.0 grams per liter of the sterilized human milk product.
51. The sterilized human milk product of any one of clauses 46-50, further comprising a concentration of 2'fucosyllactose between 0.72 grams per liter and 4.3 grams per liter of the sterilized human milk product.
52. The sterilized human milk product of any one of clauses 46-51, the product having, per 100 milliliters: (a) 63 to 67 calories, (b) 30 to 34 calories from fat, (c) 1.2 to 1.8 grams of saturated fat, (d) 10 to 16 mg of cholesterol, (e) 10 to 20 mg of sodium, (f) 4 to 10 grams of total carbohydrate, (g) 4 to 7 grams of sugars, (h) 1.2 to 1.6 grams of protein (i) 160 to 200 International Units of vitamin A, and (j) 27 to 35 mg of calcium.
53. The sterilized human milk product of any one of clauses 46-52, wherein the product has a reduced amount of *Bacillus Cereus* as compared to the amount of *Bacillus Cereus* in a raw human breast milk sample that the sterilized human milk product was sterilized from, wherein the reduced amount is at least a 500 log reduction of *Bacillus Cereus.*
54. The sterilized human milk product of any one of clauses 46-53, wherein the product has a reduced amount of *Clostridium Botulinum* as compared to the amount of *Clostridium Botulinum* in the raw human breast milk sample that the sterilized human milk product was sterilized from, wherein the reduced amount is at least a 12 log reduction of *Clostridium Botulinum.*
55. An aseptically packaged product comprising the sterilized human milk product of any one of clauses 46 - 54.
56. The aseptically packaged product of clause 55, wherein the aseptically packaged product is packaged in one of a bottle, booster cup, paper carton, paper brick, or a pouch.
57. An extended shelf life packaged product comprising the sterilized human milk product of any one of clauses 46-54.
58. The extended shelf life packaged product of clause 57, wherein the extended shelf life packaged product is packaged in one of a bottle, booster cup, paper carton, paper brick, or a pouch.

## Claims

1. A sterilized human milk product obtained from a raw human milk, the sterilized milk product comprising:
human lactoferrin, at a concentration that is above 81% of the concentration of the human lactoferrin present in the raw human milk from which the sterilized human milk product is derived, and
human immunoglobulin A, at a concentration that is above 73% of the concentration of the human immunoglobulin A present in the raw human milk from which the sterilized human milk product is derived;
wherein the sterilized human milk product has:
(i) an aerobic plate count of less than 10 CFUs per gram of the sterilized human milk product;
(ii) a *Clostridium botulinum* content that is at least 12 log reduced versus a *Clostridium botulinum* content present in the raw human milk; and
(iii) a concentration of *Bacillus cereus* that is at least 500 log lower than the concentration of *Bacillus cereus* in the raw human milk from which the sterilized human milk product is derived.

2. The sterilized human milk product of claim 1, wherein the sterilized human milk product has yeast and mold counts less than 10 CFUs per gram of the sterilized human milk product.

3. The sterilized human milk product of claim 1 or claim 2, wherein the sterilized human milk product further comprises one or more of:
human immunoglobulin G (IgG) at a concentration that is above 93% of the concentration of IgG in the raw human milk from which the sterilized human milk product is derived;
2'fucosyllactose at a concentration of between 0.72 grams per liter and 4.3 grams per liter of the sterilized human milk;
human osteopontin at a concentration that is above 87% of the concentration of osteopontin in the raw human milk from which the sterilized human milk product is derived;
human immunoglobulin M (IgM) at a concentration that is above 77% of the concentration of IgM in the raw human milk from which the sterilized human milk product is derived; and
colostrum.

4. The sterilized human milk product of any one of claims 1 to 3, wherein the sterilized milk product has one or more of:
a nitrogen utilization (NNU) protein concentration, wherein at least 95% by weight of amino acids in the NNU protein concentration are free amino acids; and
a total fat content of at least 3.0 g/100 mL of the sterilized human milk product.

5. The sterilized human milk product of claim 4, wherein the free amino acids are not bonded by a peptide bond to other amino acids.

6. The sterilized human milk product of claim 4, wherein the amino acids are in form of a L-isomer, and optionally wherein the amino acids are selected from: isoleucine, leucine, lysine, methionine, phenylalanine, threonine, tryptophan, valine and histidine.

7. The sterilized human milk product of any one of claims 1 to 6, wherein the sterilized human milk product is packaged in a pouch to confer an aseptically and extended shelf-life packaged product.

8. The sterilized human milk product of any one of claims 1 to 7, wherein the sterilized human milk product contains up to 90% less *Bacillus cereus* and *Clostridium botulinum* spores with respect to the raw human milk.

9. A commercially sterilized human milk product obtained from a raw human milk, the commercially sterilized milk product comprising:
human lactoferrin, at a concentration that is above 81% of the concentration of the human lactoferrin present in the raw human milk, and
human immunoglobulin A, at a concentration that is above 73% of the concentration of the human immunoglobulin A present in the raw human milk;
wherein the commercially sterilized human milk product has:
(i) an aerobic plate count of less than 10 CFUs per gram of the commercially sterilized human milk product;
(ii) a *Clostridium botulinum* content that is at least 12 log reduced versus a *Clostridium botulinum* content present in the raw human milk; and
(iii) a concentration of *Bacillus cereus* that is at least 500-log lower than the concentration of *Bacillus cereus* in the raw human milk from which the commercially sterilized human milk product is derived;
wherein the commercially sterilized human milk product is sterilized solely by a thermal processing.

10. The commercially sterilized human milk product of claim 9, wherein the concentration of *Bacillus cereus* is at least 1000-log lower than the concentration of *Bacillus cereus* in the raw human milk from which the commercially sterilized human milk product is derived.

11. The commercially sterilized human milk product of claim 9 or claim 10, wherein the sterilized human milk product has yeast and mold counts less than 10 CFUs per gram of the commercially sterilized human milk product.

12. The sterilized human milk product of any one of claims 1 to 8, wherein the milk product is fortified with one or more of: flavorings; vitamins; Vitamin C; Vitamin D; magnesium; and calcium.

13. A method of sterilizing human milk to produce a sterilized human milk product, the method comprising:
receiving, by a sterilizer located within a closed in-line system, as an input, a human milk sample;
flowing the human milk sample through a tube of the sterilizer in a first direction at a first flow rate;
heating the human milk sample in the tube through a countercurrent heat exchange process by flowing heating fluid in a second direction at a second flow rate, wherein the heating fluid is in contact with an exterior surface of the tube;
cooling, by the sterilizer, the heated human milk sample to produce the sterilized human milk product; and wherein heating the human milk sample in the tube comprises:
raising a temperature of the human milk sample to within a temperature range of 130° C and 150° C; and
holding the temperature of the human milk sample within the temperature range for a duration of 3 to 15 seconds.

14. A closed, in-line processing system comprising:
a sterilizer comprising:
a tube configured to flow a human milk sample through the tube in a first direction at a first flow rate; and
a container that a portion of the tube resides within, wherein the container is configured to flow a heating fluid in contact with an exterior surface of the portion of the tube in a second direction at a second flow rate such that the human milk sample flowing through the tube is heated to a predetermined temperature by countercurrent heat exchange and is held at the predetermined temperature for a duration of time.

15. A sterilized human milk product comprising:
a bacterial aerobic plate count of less than 10 CFUs per gram of the sterilized human milk product; and
a concentration of lactoferrin between 0.81 grams per liter to 13.28 grams per liter of the sterilized human milk product.
